(19) Europäisches Patentamt
European Patent Office
Office européen des brevets

(11) **EP 2 056 150 A1**

(12) **EUROPEAN PATENT APPLICATION**
published in accordance with Art. 153(4) EPC

(43) Date of publication:
**06.05.2009 Bulletin 2009/19**

(21) Application number: **07743743.2**

(22) Date of filing: **15.05.2007**

(51) Int Cl.:
***G02B 13/04*** *(2006.01)* ***A61B 1/00*** *(2006.01)*
***G02B 13/18*** *(2006.01)* ***G02B 17/08*** *(2006.01)*
***G02B 23/26*** *(2006.01)*

(86) International application number:
**PCT/JP2007/060309**

(87) International publication number:
**WO 2008/007498 (17.01.2008 Gazette 2008/03)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **10.07.2006 JP 2006188841**
**14.03.2007 JP 2007064962**

(71) Applicant: **Olympus Corporation**
**Tokyo 151-0072 (JP)**

(72) Inventor: **TOGINO, Takayoshi**
**Tokyo 151-0072 (JP)**

(74) Representative: **von Hellfeld, Axel**
**Wuesthoff & Wuesthoff**
**Patent- und Rechtsanwälte**
**Schweigerstrasse 2**
**81541 München (DE)**

# (54) TRANSMISSIVE OPTICAL ELEMENT AND OPTICAL SYSTEM USING THE SAME

(57) The invention relates to a transmitting optical element that is capable of taking images having a wide angle-of-view in general, and an image coming primarily from a vertical direction to a center axis in particular in simplified construction, is of small-format size and well corrected for aberrations, and an optical system using the same. The optical system is an imaging system that is adapted to form an object (6) around the center axis (1) on an image plane (7) orthogonal to the center axis (1). That imaging system comprises a front unit (3) rotationally symmetric about the center axis (1) and a rear unit (4) that is rotationally symmetric about the center axis (1) and has positive power. The front unit (3) comprises at least one transmitting optical element (2) formed of a transparent medium comprising two transmitting surfaces (21, 22) rotationally symmetric about the center axis (1) and having a refractive index of greater than 1. At least one transmitting surface (21, 22) of the transmitting optical element (2) is configured such that a normal thereto makes an angle with the center axis (1) near where the center axis (1) intersects the transmitting surface (21, 22).

FIG. 1

1
Y
X
Z
6
11
8
12
21
10
21
22
22
5
2
3
L1
4
L2
9
7

EP 2 056 150 A1

## Description

ART FIELD

The present invention relates generally to a transmitting optical element and an optical system using the same, and more specifically to an objective or taking optical system adapted to form an image of a portion of an object having a large angle of view on a planar form of zonal image plane.

BACKGROUNF OF THE INVENTION

Wide-angle optical systems such as fisheye lenses have so far been used as means for taking images of peripheries having a wide angle of view. However, it has still been difficult to apply them to small-format optical instruments in general and endoscopes or capsule endoscopes in particular, because a lot more optical parts are required for taking images having a wide angle of view.

So far, the inner surface of a hemispherical transparent cover at the distal end of a capsule endoscope has been configured into a conical shape to achieve a wide-angle range of observation, as proposed in Patent Publication 1. However, what principles underlie remains unclear. Patent Publication 2 has proposed configuring the front surface of the transparent cover at the distal end of a capsule endoscope into a conical shape. However, nothing is still achieved about how this transparent cover is used for a wide angle-of-view arrangement.

Patent Publication 1
JP(A) 2001-174713
Patent Publication 2
United States Patent No. 5,604,531

DISCLOSURE OF THE INVENTION

Such being the situations of the prior art, a main object of the invention is to provide a transmitting optical element that is of small-format size and well corrected for aberrations, and is capable of taking images having a wide angle of view in general, and images coming primarily from the vertical direction with respect to a center axis in particular, in simplified construction, and an optical system using the same.

According to the invention, the above object is accomplishable by the provision of a transmitting optical element comprising a transparent medium including two transmitting surfaces rotationally symmetric about a center axis and having a refractive index of greater than 1, **characterized in that** at least one transmitting surface is configured such that a normal thereto makes an angle with the center axis near where the center axis intersects said transmitting surface.

The invention also provides an optical system that is an imaging system adapted to form an object about a center axis on a planar image plane orthogonal to the center axis, characterized by comprising a front unit rotationally symmetric about the center axis and a rear unit that is rotationally symmetric about the center axis and has positive power, wherein said front unit comprises at least one transmitting optical element formed of a transparent medium comprising two transmitting surfaces rotationally symmetric about the center axis and having a refractive index of greater than 1, wherein at least one transmitting surface of said transmitting optical element is configured such that a normal thereto makes an angle with the center axis near where the center axis intersects said transmitting surface.

In this case, it is desired that between said transmitting optical element and said rear unit or in said rear unit, there be an aperture provided coaxially to the center axis.

It is also desired that any of said transmitting surfaces of said transmitting optical element be configured in such a way as to be concave with respect to said aperture.

Further, it is desired to satisfy the following condition (1):

$$1^\circ < \theta < 90^\circ \quad \cdots (1)$$

where tangents of the two transmitting surfaces of said transmitting optical element make an angle $\theta$ (°) at a point where a chief ray strikes a first transmitting surface and a point where a chief ray strikes a second transmitting surface with the proviso that the chief ray is defined by a ray traveling from a center of an angle of view in a direction along the center axis through a center of said aperture.

Still further, it is desired that at least said transmitting optical element located in said front unit and nearest to an object side comprises a light block member adapted to block off a ray from on the center axis.

Yet further, it is desired that said rear unit comprise a rotationally symmetric optical system concentric with the center axis.

Yet further, it is desired that said rear unit having positive power is adapted to correct chromatic aberration of magnification occurring from said front unit.

Yet further, it is desired to form a cylindrical or spherical image in a full-360° direction on a two-dimensional imaging device located on said image plane.

Yet further, it is desired that said transmitting surface configured such that the normal thereto makes an angle with the center axis near where it intersects the center axis have a rotationally symmetric shape that is formed by rotation about the center axis of a curved line of any desired shape having no plane of symmetry.

Yet further, it is desired that said transmitting surface configured such that the normal thereto makes an angle with the center axis near where it intersects the center axis have a rotationally symmetric shape that is formed by rotation about the center axis of a curved line of any desired shape including an odd number order term.

The invention also encompasses an optical system used as the one to project an image located on an image plane onto an object plane.

According to the invention as described above, it is possible to obtain a transmitting optical element that is of small-format size and well corrected for aberrations, has high resolving power, and is capable of taking or project a wide angle-of-view image in simplified construction.

BRIEF DESCRIPTION OF THE DRAWINGS

Fig. 1 is a sectional view of the optical system according to Example 1 of the invention, as taken along its center axis.
Fig. 2 is a transverse aberration diagram for the optical system of Example 1.
Fig. 3 is illustrative of the object height vs. image height relations of Example 1 in the meridional section.
Fig. 4 is a sectional view of one modification to the transmitting optical element in Example 1.
Fig. 5 is a sectional view of the optical system according to Example 2 of the invention, as taken along its center axis.
Fig. 6 is a transverse aberration diagram for the optical system of Example 2.
Fig. 7 is illustrative of the object height vs. image height relations of Example 2 in the meridional section.
Fig. 8 is a sectional view of one modification to the transmitting optical element in Example 2.
Fig. 9 is a sectional view of another modification to the transmitting optical element in Example 2.
Fig. 10 is a sectional view of the optical system according to Example 3 of the invention, as taken along its center axis.
Fig. 11 is a transverse aberration diagram for the optical system of Example 3.
Fig. 12 is illustrative of the object height vs. image height relations of Example 3 in the meridional section.
Fig. 13 is a sectional view of the optical system according to Example 4 of the invention, as taken along its center axis.
Fig. 14 is a transverse aberration diagram for the optical system of Example 4.
Fig. 15 is illustrative of the object height vs. image height relations of Example 4 in the meridional section.
Fig. 16 is a sectional view of the optical system according to Example 5 of the invention, as taken along its center axis.
Fig. 17 is a transverse aberration diagram for the optical system of Example 5.
Fig. 18 is illustrative of the object height vs. image height relations of Example 5 in the meridional section.
Fig. 19 is a sectional view of the optical system according to Example 6 of the invention, as taken along its center axis.
Fig. 20 is a transverse aberration diagram for the optical system of Example 6.
Fig. 21 is illustrative of the object height vs. image height relations of Example 6 in the meridional section.
Fig. 22 is a sectional view of the optical system according to Example 7 of the invention, as taken along its center axis.
Fig. 23 is a transverse aberration diagram for the optical system of Example 7.
Fig. 24 is illustrative of the object height vs. image height relations of Example 7 in the meridional section.
Fig. 25 is illustrative of an example of using the inventive wide angle-of-view taking optical system as a taking optical system at the distal end of an endoscope.
Fig. 26 is illustrative of one example of using the inventive wide angle-of-view taking optical system as a taking optical system for a capsule endoscope.

BEST MODE FOR CARRYING OUT THE INVENTION

The transmitting optical element and optical system of the invention are now explained with reference to some specific examples.

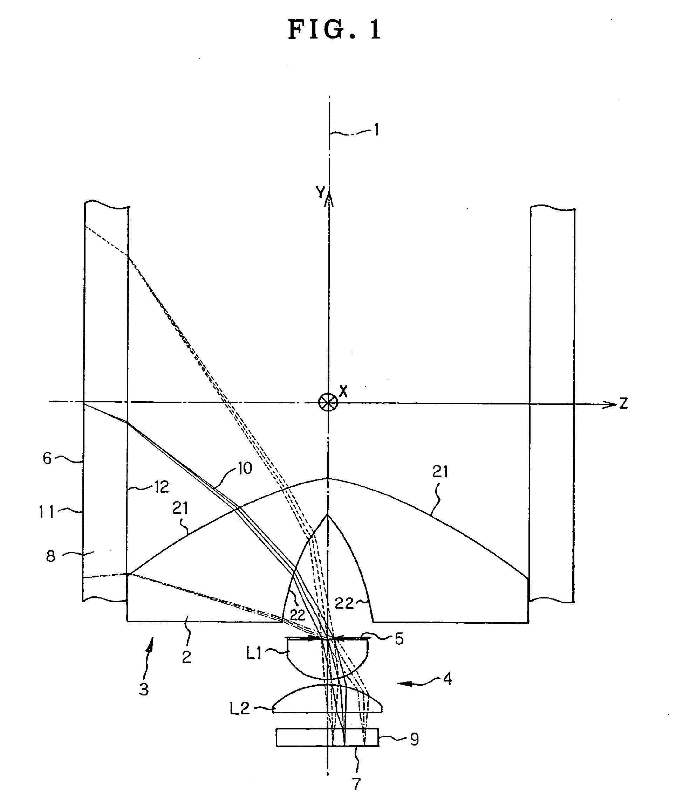

Fig. 1 is a sectional view of the optical system according to Example 1, as taken along the center axis 1 (the axis of rotational symmetry). The optical system of the invention is now explained more specifically with reference to an imaging optical system adapted to form an image on an object plane 6 rotationally symmetric about the center axis 1 on a planar image plane 7 orthogonal to the center axis 1. Note, however, that by reversing the optical path taken, the inventive optical system may just as well be used in the form of a projection optical system wherein the image plane 7 is projected onto the object plane 6.

First of all, the inventive transmitting optical element and optical system are explained with reference to Example 1 of Fig. 1. The optical system of Fig. 1 is built up of a front unit 3 comprising at least one transmitting optical element 2 having a rotationally symmetric shape about the center axis 1, and a rear unit 4 that is located on an image plane 7 side with respect to the front unit 3, comprises an ordinary rotationally symmetric optical system, is rotationally symmetric about the center axis 1 and has positive power. Between the front unit 3 and the rear unit 4 or in the rear unit 4, there is an aperture stop 5 located coaxially to the center axis 1.

In the example here, one transmitting optical element 2 that is an element constituting the front unit 3 has two transmitting surfaces 21 and 22. More specifically, that surfaces 21 and 22 are each constructed of an extended rotation free-form surface defined by the rotation about the center axis 1 of a curved line located at a position off the center axis 1 in a section including the center axis 1 (the paper plane of Fig. 1), and the transmitting surfaces 21 and 22 are each configured such that the normal to it makes an angle with the center axis 1 near the center axis 1. Note here that the extended rotation free-form surface will be described later.

Thus, the transmitting optical element 2 in the front unit 3 comprises at least one transmitting surface 21, 22 constructed of an extended rotation free-form surface, and is configured such that the normal to the transmitting surface 21, 22 makes an angle with the center axis 1 near where it intersects the center axis 1; in a section (meridional section) including the center axis 1, a light beam from an image around the center axis 1 is converted by strong wedge effect into a light beam from an image in front of (along the center axis 1) the rear unit 4. The rear unit 4 works as an optical system for projecting a front image (real or virtual image) having an angle of view converted by the front unit 3 onto the image plane 7 as a zonal image.

It is here important that the transmitting optical element 2 working as the angle-of-view conversion means in the front unit 3 be of shape obtained by the rotation about the center axis 1 of a substantial wedge whose thickness in the meridional section grows large with an increasing distance from the center axis 1 to the periphery. This wedged refraction is located rotationally symmetrically about the center axis 1 acting as the center of rotation so that a light beam coming from a direction nearly orthogonal to the center axis 1 can reasonably be guided to the rear unit 4.

With a conventional fisheye lens or the like, on the other hand, similar effects are achievable by locating a lens of similar tight negative meniscus shape at the first surface. However, when there is no particular need of having imaging capability on the center axis 1, the surface shape is not necessarily of continuity near the center axis 1; indeed, peripheral refraction is more easily obtainable with a discontinuous surface shape.

In the example here, therefore, the two transmitting surfaces 21 and 22 are each provided in a discontinuous surface form at the position of the center axis 1.

If the aperture stop 5 coaxial to the center axis and an optical system having positive power (the rear unit 4) are located on the image side of the transmitting optical element 2, it is then possible to take or project images of good contrast, because only a light beam limited by the aperture stop 5 is allowed to enter the rear unit 4.

More importantly or more preferably, the two transmitting surfaces 21 and 22 of the transmitting optical element 2 be each of shape concave with respect to the aperture stop 5 for the purpose of holding back the occurrence of coma.

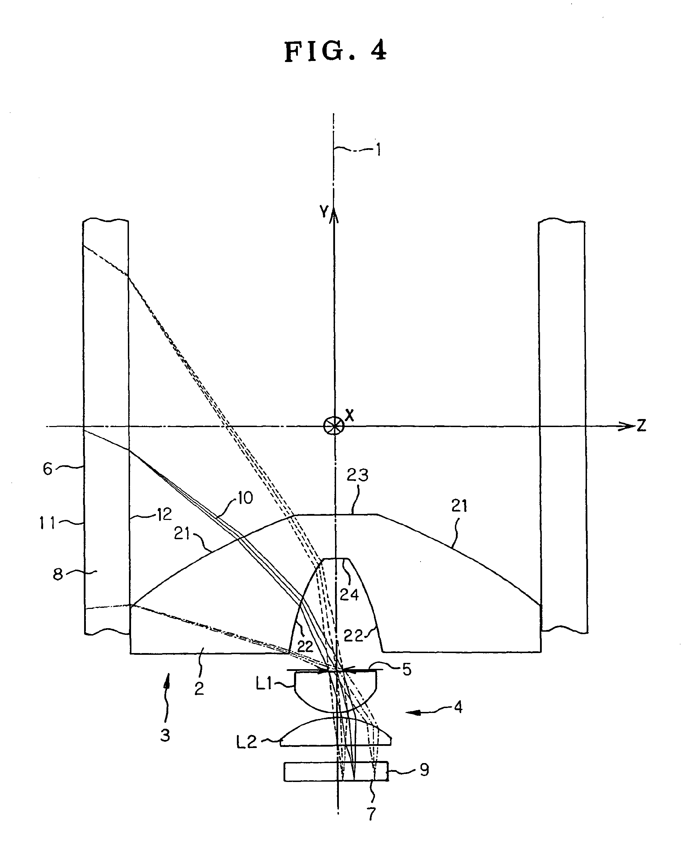

More preferably, areas of the transmitting surfaces 21 and 22 near the center axis 1 are formed of discontinuous optical surfaces such as mutually parallel planes 23 and 24, etc., as shown in Fig. 4, because images in the center axis 1 direction, too, can be taken by forming them on the image plane 7.

It is more preferable to satisfy the following condition (1):

$$1^\circ < \theta < 90^\circ \quad \cdots (1)$$

where the tangents of the two transmitting surfaces of the transmitting optical element make an angle $\theta$ (° ) at a point where a chief ray 10 strikes the first transmitting surface 21 located in the transmitting optical element 2 and nearest to the object side and a point where the chief ray 10 strikes the second transmitting surface 22 with the proviso that the chief ray 10 is defined by a ray that travels from the center of an angle of view through the center of the aperture stop 5.

As the lower limit of 1° to the above condition (1) is not reached, the amount of conversion of the angle of view at the transmitting optical element 2 becomes too small to obtain a wide peripheral visual field. As the upper limit of 90° is exceeded, the amount of conversion of the angle of view grows too large, producing too much chromatic aberrations to be corrected at other surface.

When such a large angle of conversion is needed, it is more preferable to use two or more sets of the inventive transmitting optical elements.

More preferably,

$$10^\circ < \theta < 60^\circ \quad \cdots (1\text{-}1)$$

Set out below are the values of θ in Examples 1 to 7 given later.

| | Example 1 | Example 2 | Example 3 | Example 4 |
|---|---|---|---|---|
| θ | 44.60° | 45.79° | 58.62° | 34.215° |

| | Example 5 | Example 6 | Example 7 |
|---|---|---|---|
| θ | 33.667° | 30.962° | 25.8923° |

More preferably, the rear unit 4 having positive power is constructed from an optical system that is rotationally symmetric about and concentric with the center axis 1, because the transmitting optical element 2 can be used as an attachment optical system for ordinary optical systems.

More preferably, the transmitting optical element 2, because of having the strong wedge action, is fabricated from a glass material having reduced or limited dispersion. However, the glass having reduced dispersion (or a large Abbe constant) has a relatively low refractive index, and causes chromatic aberrations occurring at the transmitting optical element 2 to grow too large for correction. In such cases, if the chromatic aberrations produced at the transmitting optical element 2 are corrected at the rear unit 4, it is then possible to increase resolving power. The chromatic aberrations occurring primarily at the transmitting optical element 2 are chromatic aberration of magnification so that images having totally reduced chromatic aberrations and high resolving power are achievable by correcting the size of an electronically captured image in the case of a taking optical system, and by making previous correction of the size of an image on the display device for each color in the case of a projection optical system.

More preferably in the example here, the wedge effect should be obtained by use of the rotationally symmetric surface shape. Besides, such wedge effect may just as well be obtained by use of transmitting surfaces acting to bend light rays (electromagnetic waves), for instance, HOEs, DOEs, inhomogeneous media or Fresnel lenses. In that case, it is important to make the bending action discontinuous near the center axis 1. In other words, use may be made of every surface serving to change the angle made between the incident rays and the center axis 1 from large to small.

Further, the optical system of the invention comprises such transmitting optical element 2, the aperture stop 5 located coaxially to the center axis 1, and the rear unit 4 having coaxial positive power and collecting action.

More preferably, at the transmitting surfaces 21 and 22 of the transmitting optical element 2, there is astigmatism produced, because of a difference in power between the sagittal section (that is orthogonal to the meridional section and includes the chief ray 10) and the meridional section. The ensuing astigmatism is rotationally symmetric one but not simple one as produced in a rotationally symmetric system. Therefore, it is desired that an aspheric surface having a function of correcting that astigmatism be located near the image plane 7.

More preferably, an extended rotation free-form surface having different powers in the sagittal and meridional sections should be located as the astigmatism correction surface near the image plane.

Furthermore, if rotationally symmetric surfaces formed by the rotation about the center axis 1 of a curved line of any desired shape having no plane of symmetry are used for the transmitting surfaces 21 and 22 of the transmitting optical element 2, it is then possible to make correction of distortion around the peripheral portion of the angle of view.

It is desired that at least the aforesaid transmitting optical element 2 located in the front unit 3 and nearest to the object side further comprise a light block member adapted to block off light rays coming from on the center axis 1. None of imaging light from the object plane 6 is incident near the center axis 1 of the transmitting optical element 2: if that position is blocked by the light block member, it is then possible to prevent inessential light such as flare light from entering the optical system.

More preferably, that curved line should be of any desired shape including an odd order term. That odd order term is preferable for correction of aberrations, because of giving vertically asymmetric shape to the center of the angle of view.

The optical system of the invention is now explained more specifically with reference to Examples 1 to 7. The constructional parameters in these examples, which will be given later, have been determined on the results of normal ray tracing from the object plane 6 to the image plane 7 via the front unit 3 and the rear unit 4, as shown typically in Fig. 1.

For a coordinate system, assume that, in normal ray tracing as shown typically in Fig. 1, the origin of a decentrated optical surface of a decentrated optical system is defined by a point (Examples 1 and 2) where the point at which the chief ray 10 intersects the object plane 6 is projected onto the axis of rotational symmetry (the center axis) 1 or a point (Examples 3 to 7) where the point at which the chief ray 10 intersects the first transmitting surface 21 of the transmitting optical element 2 is projected onto the axis of rational symmetry (the center axis) 1, the Y-axis positive direction is defined by a direction of the axis of rotational symmetry (the center axis) 1 away from the image plane 7, and the Y-Z plane is defined by the paper plane of Fig. 1. And, the Z-axis positive direction is defined by a direction on a side opposite to the object side now in consideration, and the X-positive positive direction is defined by an axis that forms with the Y- and Z-axes a right-handed orthogonal coordinate system.

Given for the decentered surface are the amount of decentration of that surface from the center of the origin of the aforesaid optical system on a coordinate system on which that surface is defined (X, Y and Z are indicative of the X-axis direction, the Y-axis direction, and the Z-axis direction, respectively), and the angles of tilt ($\alpha$, $\beta$, $\gamma$ (° )) of the coordinate systems for defining the surfaces with the centers on the X-, Y- and Z-axes, respectively. In that case, the positive for $\alpha$ and $\beta$ means counterclockwise rotation with respect to the positive directions of the respective axes, and the positive for $\gamma$ means clockwise rotation with respect to the positive direction of the Z-axis. Referring here to how to perform $\alpha$-, $\beta$- and $\gamma$- rotations of the center axis of the surface, the coordinate system that defines each surface is first $\alpha$-rotated counterclockwise about the X-axis of the coordinate system that is defined at the origin of the optical system. Then, the coordinate system is $\beta$-rotated counterclockwise about the Y-axis of the rotated new coordinate system. Finally, the coordinate system is $\gamma$-rotated clockwise about the Z-axis of the rotated new another coordinate system.

When, of optical surfaces forming the optical system of each example, a specific surface and the subsequent surface form together a coaxial optical system, there is a surface spacing given. Besides, the radius of curvature of each surface and the refractive index and Abbe constant of the medium are given as usual.

It is noted that the term with respect to aspheric surfaces on which no data are mentioned in the constructional parameters, given later, is zero. Refractive indices and Abbe constants are given on a d-line (587.56 nm wavelength) basis, and length in mm. The decentration of each surface is given in terms of the amount of decentration from the origin of the aforesaid optical system, as described above.

In this conjunction, the extended rotation free-form surface is a rotationally symmetric surface given by the following definition.

First, the following curve (b) passing through the origin on the Y-Z coordinate plane is determined.

$$
\begin{aligned}
Z = {} & (Y^2/RY)/[1+\{1-(C_1+1)Y^2/RY^2\}^{1/2}] \\
& C_2Y+C_3Y^2+C_4Y^3+C_5Y^4+C_6Y^5+C_7Y^6+ \cdots +C_{21}Y^{20}+ \cdots \\
& C_{n+1}Y^n+ \cdots \qquad \text{(b)}
\end{aligned}
$$

Then, a curve F(Y) is determined by the rotation through an angle $\theta$ (° ) of that curve (b) in the X-axis positive direction provided that the counterclockwise direction is taken as positive. This curve F(Y), too, passes through the origin on the Y-Z coordinate plane.

That curve F(Y) is parallel translated by a distance R in the Z-positive direction (in the Z-negative direction when R is negative), and the parallel translated curve is then rotated about the Y-axis to generate a rotationally symmetric surface by which the extended rotation free-form surface is defined.

As a result, the extended rotation free-form surface becomes a free-form surface (free-form curve) in the Y-Z plane, and a circle with a radius |R| in the X-Z plane.

From this definition, the Y-axis becomes the axis (the axis of rotational symmetry) of the extended rotation free-form surface.

Here, RY is the radius of curvature of the spherical term in the Y-Z section, $C_1$ is a conical constant, and $C_2$, $C_3$, $C_4$, $C_5$, etc. are the aspheric coefficients of first, second, third, and fourth order, respectively.

For the inventive optical system, it is desired that at least one transmitting surface in the front unit 3 be constructed of such an extended rotation free-form surface. By allowing at least one transmitting surface to have such surface shape, it is possible to give strong wedge action to the front unit 3 in the meridional section, thereby taking or projecting wide angle-of-view images.

Example 1

Fig. 1 is illustrative in section of the optical system of Example 1 as taken along its center axis 1 (the axis of rotational symmetry).

The optical system here is built up of the front unit 3 comprising the transmitting optical element 2 formed of a transparent medium that is rotationally symmetric about the center axis 1 and has a refractive index of greater than 1, and the rear unit 4 that is located on the image plane 7 side with respect to the front unit 3, comprises a lens system consisting of two groups or two lenses, is rotationally symmetric about the center axis 1 and has positive power, with the aperture stop 5 being interposed between the front unit 3 and the rear unit 4 in a coaxial relation to the center axis 1. The first transmitting surface 21 of the transmitting optical element 2 is constructed from an extended rotation free-form surface of pointed shape that is obtained by the rotation about the center axis 1 as the axis of rotational symmetry of a curved line defined by an arc having a center at a position off the center axis 1 in a section including the center axis 1, and the second transmitting surface 22, too, is constructed from an extended rotation free-form surface of pointed shape that is obtained by the rotation about the center axis 1 as the axis of rotational symmetry of a curved line defined by an arc having a center at a position off the center axis 1, both the first 21 and the second transmitting surface 22 being of a shape concave with respect to the aperture stop 5. And the first 21 and the second transmitting surface 22 are each configured such that the normal makes an angle with the center axis 1 near where it intersects the center axis 1, and each of a substantial wedge shape in the section including the center axis 1, whose thickness in the meridional section grows large with an increasing distance from the center axis 1 to the periphery.

And in the example here, the object plane 6 is comprised of an outer cylindrical surface 11 and an inner cylindrical surface 12, both concentric with the center axis 1, and is in alignment with the outer cylindrical surface 11 of the protective transparent cylinder 8 for the optical system according to the example here.

The rear unit 4 is made up of a plano-convex positive lens L1 and a convex-plano positive lens L2, and the aperture stop 5 is located just before the plano-convex positive lens L1. And, the image plane 7 of this optical system is in alignment with the back surface of the plane-parallel plate 9.

Note here that the transparent cylinder 8 and the plane-parallel plate 9 may be dispensed with.

Such being the arrangement, an object image on the cylindrical object plane 6, having a wide angle of view of ±4 mm, is formed as a zonal image on the planar image plane 7 vertical to the center axis 1 via the transmitting optical element 2 rotationally symmetric about the center axis 1, the stop 5 and the lens system constituting the rear unit 4.

The specifications of Example 1 are:

Object height: ±4 mm,
Numerical aperture (NA): 0.1, and
Image size: ϕ0.26 to ϕ1.64 mm.

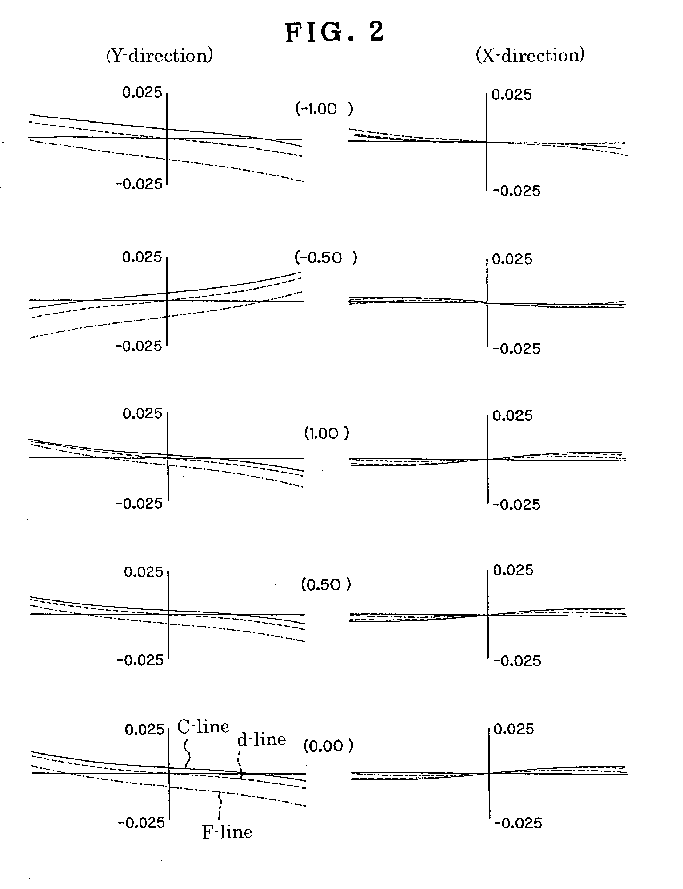

Transverse aberrations of the optical system here are shown in Fig. 2, wherein the figures at the center stand for relative image heights, and Y (meridional)- and X (sagittal)-direction transverse aberrations at those object heights are shown. The same shall apply to Example 2, too.

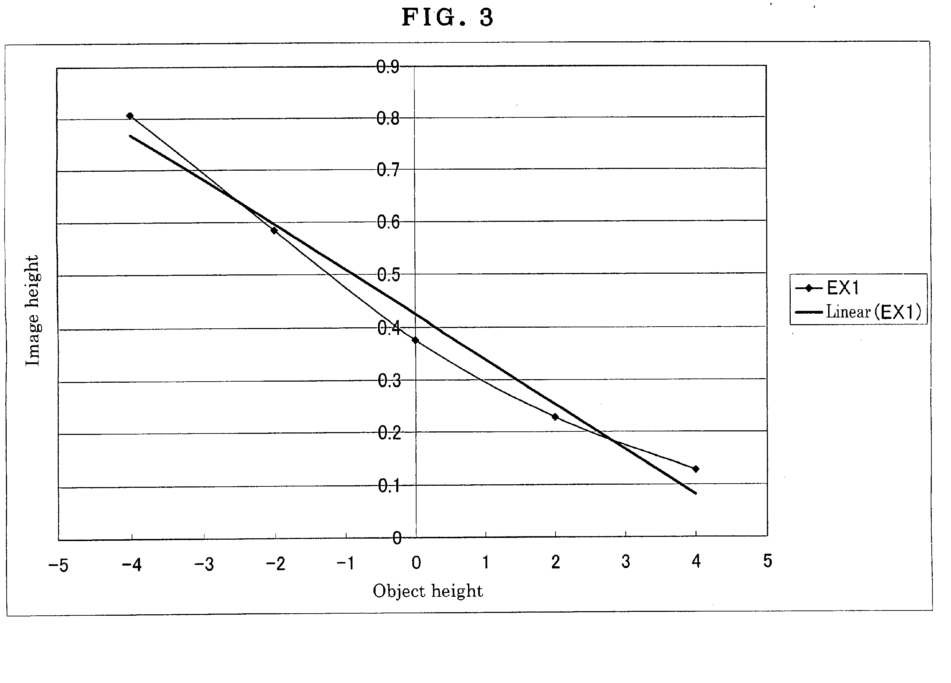

Object height (mm) vs. image height (mm) relations in the meridional section here are shown in Fig. 3, wherein a linear line is indicative of where the object height and image height are in a linear relation. The same shall apply to Example 2, too.

Example 2

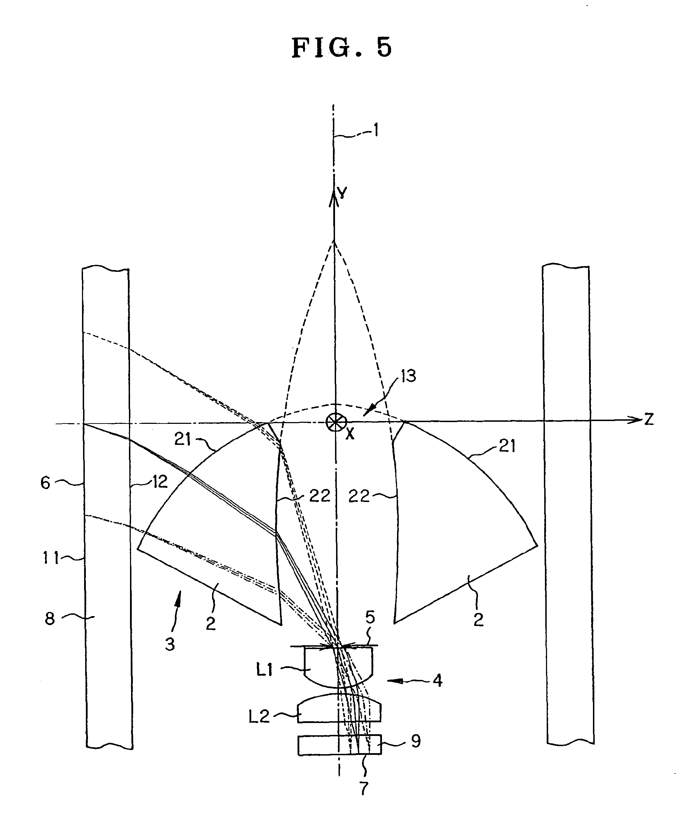

Fig. 5 is illustrative in section of the optical system of Example 2 as taken along its center axis 1 (the axis of rotational symmetry).

The optical system here is basically the same as in Example 1, with the exception that the transmitting optical element 2 has the hole 13 formed in its center. More specifically, the optical system is built up of the front unit 3 comprising the transmitting optical element 2 formed of a transparent medium that is rotationally symmetric about the center axis 1 and has a refractive index of greater than 1, and the rear unit 4 that is located on the image plane 7 side with respect to the front unit 3, comprises a lens system consisting of two groups or two lenses, is rotationally symmetric about the center axis 1 and has positive power, with the aperture stop 5 being interposed between the front unit 3 and the rear unit 4 in a coaxial relation to the center axis 1. The first transmitting surface 21 of the transmitting optical element 2 is constructed from an extended rotation free-form surface of pointed shape that is obtained by the rotation about the center axis 1 as the axis of rotational symmetry of a curved line defined by an arc having a center at a position off the center axis 1 in a section including the center axis 1, and the second transmitting surface 22, too, is constructed from an extended rotation free-form surface of pointed shape that is obtained by the rotation about the center axis 1 as the axis of rotational symmetry of a curved line defined by an arc having a center at a position off the center axis 1, both the first 21 and the second transmitting surface 22 being of a shape concave with respect to the aperture stop 5. And the first 21 and the second transmitting surface 22 are each configured such that the normal makes an angle with the center axis 1 near where it intersects the center axis 1, and each of a substantial wedge shape in the section including the center axis, whose thickness in the meridional section grows large with an increasing distance from the center axis 1 to the periphery. Further, the transmitting surfaces 21 and 22 intersect near the center axis 1, and the hole 13 with the center axis 1 as center is centrally formed through them.

And in the example here, the object plane 6 is comprised of an outer cylindrical surface 11 and an inner cylindrical surface 12, both concentric with the center axis 1, and is in alignment with the outer cylindrical surface 11 of the protective transparent cylinder 8 for the optical system according to the example here.

The rear unit 4 is made up of a plano-convex positive lens L1 and a convex-plano positive lens L2, and the aperture stop 5 is located just before the plano-convex positive lens L1. And, the image plane 7 of this optical system is in alignment with the back surface of the plane-parallel plate 9.

Note here that the transparent cylinder 8 and the plane-parallel plate 9 may be dispensed with.

Such being the arrangement, an object image on the cylindrical object plane 6, having a wide angle of view of ±2 mm, is formed as a zonal image on the planar image plane 7 vertical to the center axis 1 via the transmitting optical element 2 rotationally symmetric about the center axis 1, the stop 5 and the lens system constituting the rear unit 4.

The specifications of Example 2 are:

Object height: ±2.00 mm,
Numerical aperture (NA): 0.01, and
Image size: ϕ0.45 to ϕ1.26 mm.

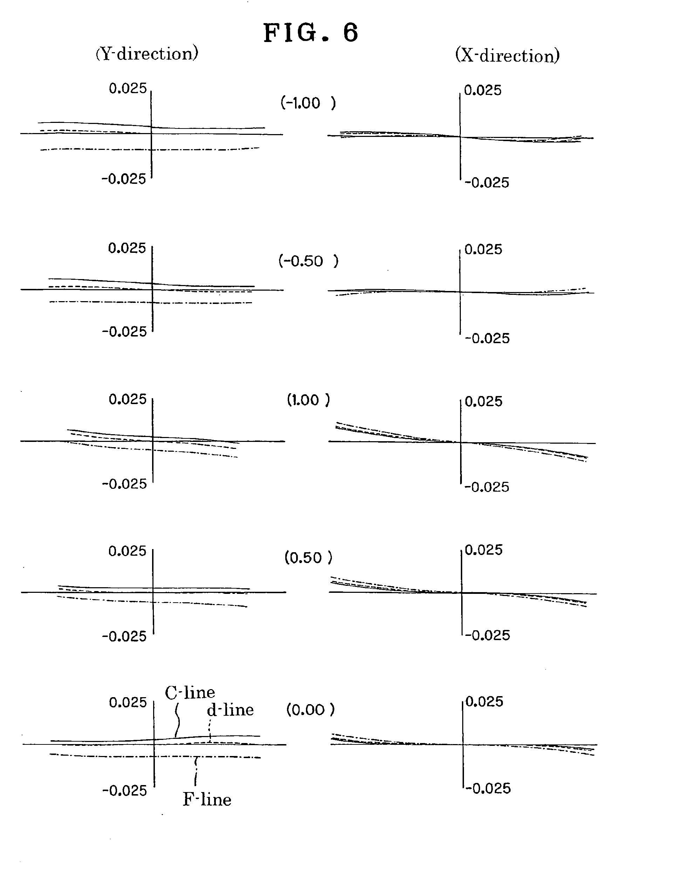

Transverse aberrations of the optical system as in Fig. 2 here are shown in Fig. 6.

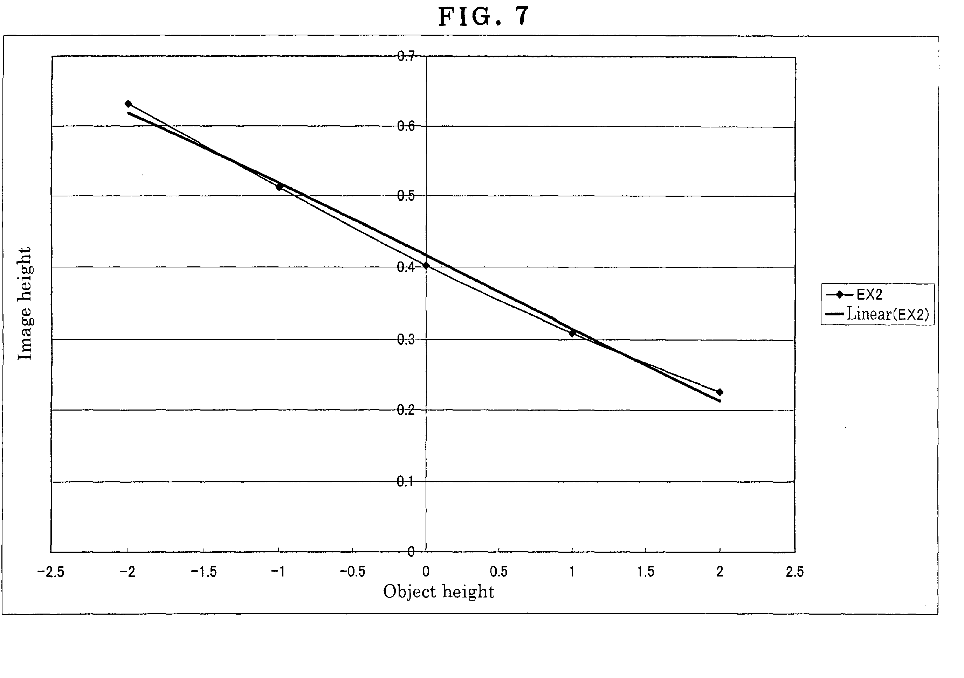

Object height (mm) vs. image height (mm) relations in the meridional section here are shown in Fig. 7.

In the example here, the second transmitting surface 22 of the transmitting optical element 2 is located parallel with the center axis 1, and configured in a planar symmetric form with respect to the plane orthogonal to the center axis 1.

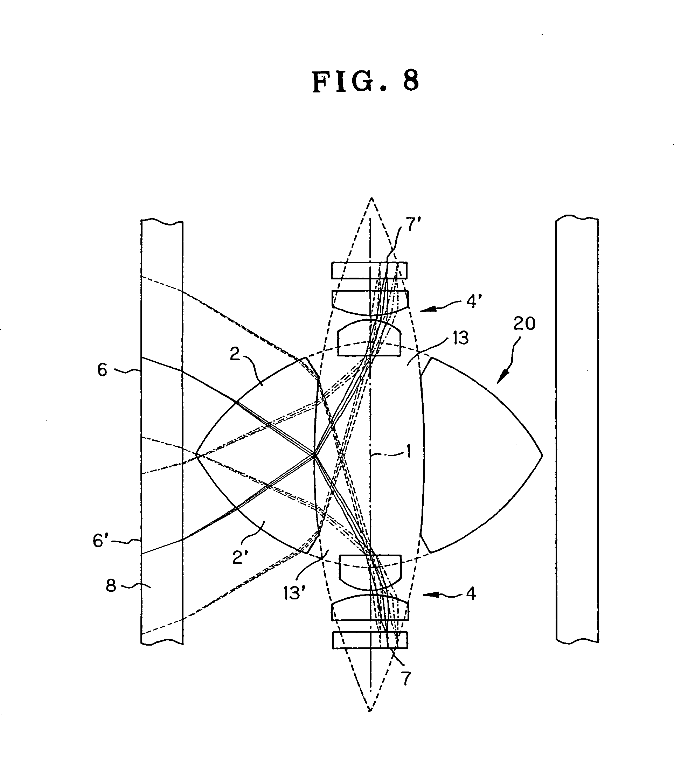

One modification to Example 2 is shown in section in Fig. 8. Two optical systems according to the example here are located plane symmetrically with respect to the plane orthogonal to the center axis 1. In that case, the transmitting optical element 2 in one optical system and the transmitting optical element 2' in another are set up as one common transmitting optical element 20. Then, the rear units 4, 4' and image planes 7, 7' are located above and below a direction along the center axis 1 of that transmitting optical element 20 so that images on the object planes 6, 6' positioned vertically in parallel and in alignment with the outer cylindrical surface of the common transparent cylinder 8 are formed on the respective image planes 7, 7', ensuring that a wide object plane can simultaneously be covered.

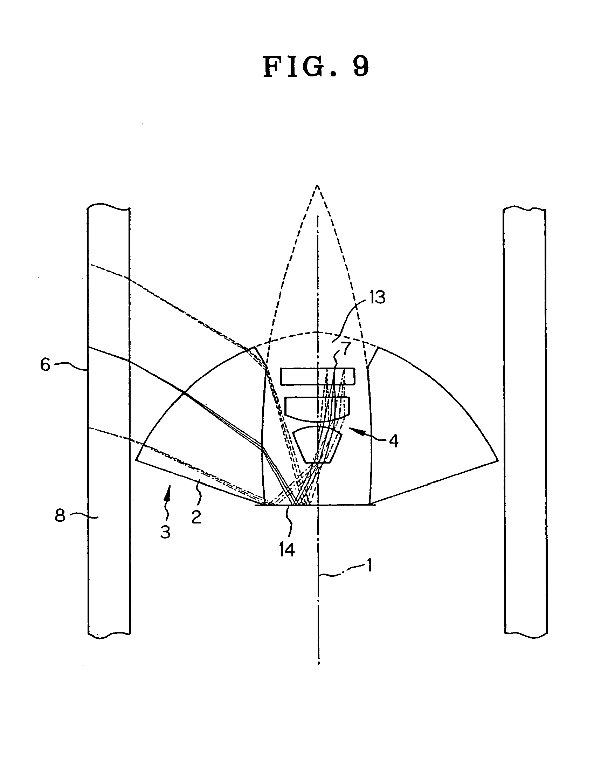

Another modification to Example 2 is shown in section in Fig. 9. In this modification, the plane mirror 14 orthogonal to the center axis 1 is interposed between the front and rear units 3 and 4 in the optical system to bend back an optical path, and the rear unit 4 and the image plane 7 are located in the hole 13 centrally formed through the transmitting optical element 2, thereby achieving a further total size reduction.

Example 3

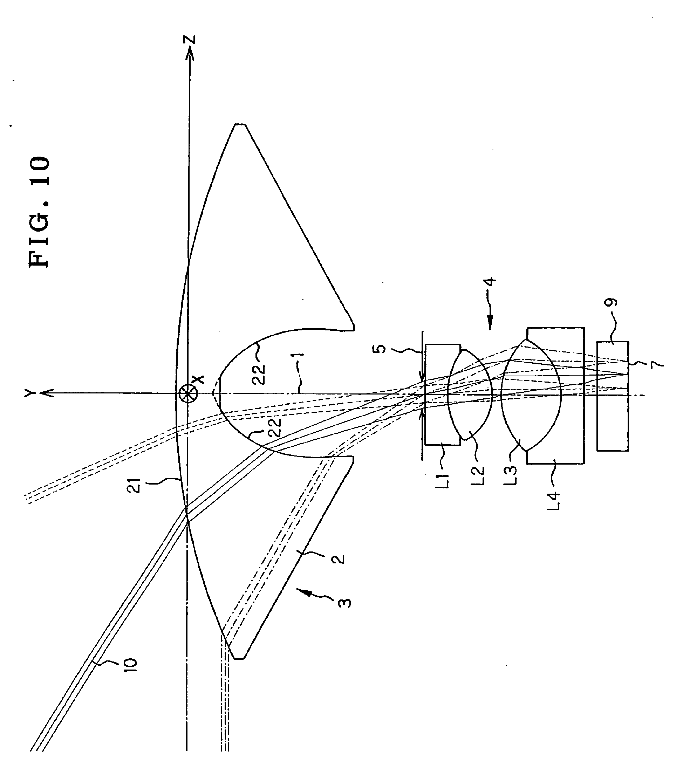

Fig. 10 is a sectional view of the optical system of Example 3 as taken along the center axis (the axis of rotational symmetry) 1.

In the optical system here, the first transmitting surface 21 of the transmitting optical element 2 is constructed of a convex spherical surface having a center on the center axis 1, and only the second transmitting surface 22 is constructed from an extended rotation free-form surface of pointed shape that is obtained by the rotation about the center axis 1 of a curved line having a higher order term, with the rear unit 4 consisting of two groups or four lenses. More specifically, the optical system here is built up of the front unit 3 comprising the transmitting optical element 2 formed of a transparent medium that is rotationally symmetric about the center axis 1 and has a refracting index of greater than 1, and the rear unit 4 that is located on the image plane 7 side with respect to the front unit 3, comprises a lens system consisting of two groups or four lenses, is rotationally symmetric about the center axis 1 and has positive power, with the aperture stop 5 interposed between the front unit 3 and the rear unit 4 coaxially to the center axis 1. The first transmitting surface 21 of the transmitting optical element 2 is formed of a spherical surface that has a center on the center axis 1 and is convex in the Y-axis positive direction, and the second transmitting surface 22 is constructed from an extended rotation free-form surface of pointed shape that is obtained by the rotation about the center axis 1 as the axis of rotational symmetry of a curved line with the Y third-order term and fourth-order term added to the spherical term, both the first and second transmitting surfaces 21 and 22 being of a shape concave with respect to the aperture stop 5. And the second transmitting surface 22 is configured such that the normal makes an angle with the center axis 1 near where it intersects the center axis 1, and in the section including the center axis 1, the transmitting optical element 2 is configured in a substantial wedge form whose thickness in the meridional section grows large with an increasing distance from the center axis 1 to the periphery.

And in the example here, the object plane 6 is set at infinity in the Z-axis minus direction.

The rear unit 4 is made up of a cemented lens of a plano-concave negative lens L1 and a double-convex positive lens L2 and a cemented lens of a double-convex positive lens L3 and a concave-plano negative lens L4, and the aperture stop 5 is located just before the plano-concave negative lens L1. And the image plane 7 of this optical system is in alignment with the back surface of the plane-parallel plate 9. Note here that the plane-parallel plate 9 may be dispensed with.

Such being the arrangement, an object image, having a wide angle of view of 65° at infinity, is formed as a zonal image on the planar image plane 7 vertical to the center axis 1 via the transmitting optical element 2 rotationally symmetric about the center axis 1, the stop 5 and the lens system constituting the rear unit 4.

The specifications of Example 3 are:

Angle-of-view range: 65° (with the center angle of view of 32.5° ),
Entrance pupil diameter: 0.16, mm, and
Image size: $\phi$0.20 to $\phi$0.91 mm.

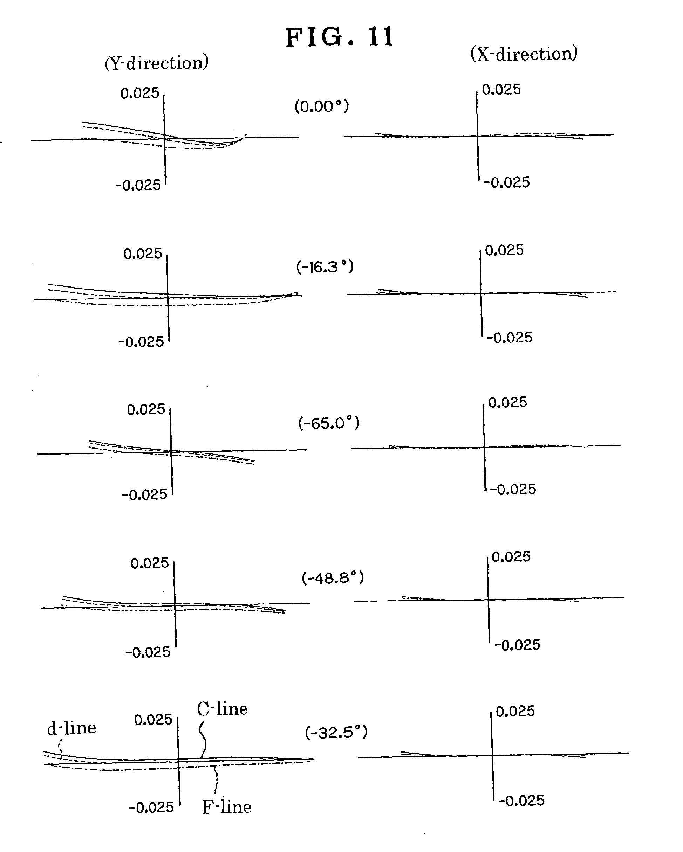

Transverse aberrations of the optical system here are shown in Fig. 11, wherein the angles given at the center are indicative of angles of view as measured from the plane orthogonal to the center axis 1, and transverse aberrations at those angles of view in the Y (meridional) and X (sagittal) directions are shown. The same shall apply to Examples 4 to 7, too.

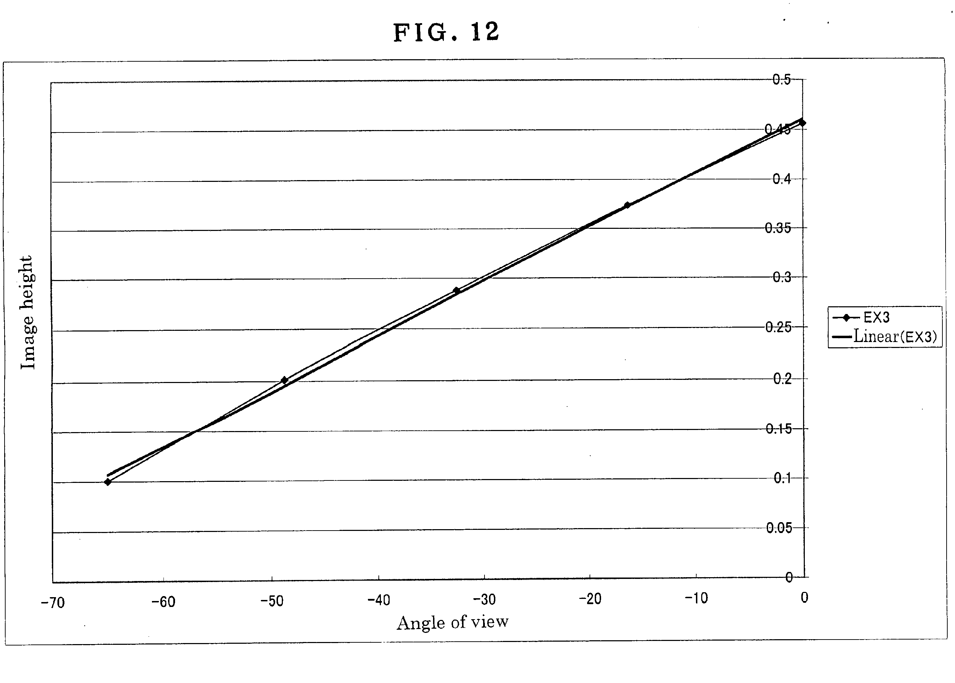

Angles of view vs. image height (mm) relations in the meridional section here are shown in Fig. 12, wherein a linear line is indicative of where the angle of view and the image height are in linear relations. The same shall apply to Examples 4 to 7, too.

Note here that if another optical surface or system is introduced in an area of the transmitting surface 21, 22 near the center axis 1, it is also possible to form and take images in the center axis 1 direction on the image plane 7, thereby taking images having a wider angle of view.

Example 4

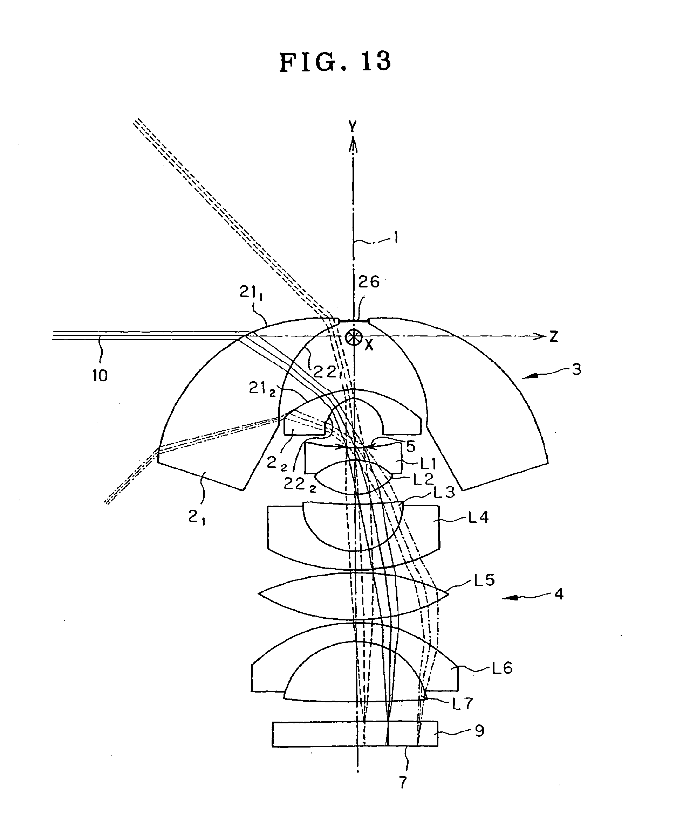

Fig. 13 is a sectional view of the optical system of Example 4 as taken along the center axis (the axis of rotational symmetry) 1.

In the optical system here, two transmitting optical elements $2_1$ and $2_2$ formed of a transparent medium having a refractive index of greater than 1 are used for the front unit 3. The first and second transmitting surfaces $21_1$ and $22_1$ of the transmitting optical element $2_1$, and the first and second transmitting surfaces $21_2$ and $22_2$ of the transmitting optical element $2_2$ are each constructed from an extended rotation free-form surface, i.e., a toric surface with the center axis 1 as the axis of rotational symmetry, which is obtained by the rotation about the center axis 1 as the axis of rotational symmetry of a curved line having only the spherical term free of any higher order term. The transmitting surfaces $21_1$, $22_1$, $21_2$ and $22_2$ are each configured such that it has a convex shape on its object side at a position where the chief ray 10 is incident and in the section including the center axis 1. The rear unit 4 that is located on the image plane 7 side with respect to the front unit 3, is rotationally symmetric about the center axis 1 and has positive power consists of four groups or seven lenses. And the aperture stop 5 is interposed between the front unit 3 and the rear unit 4 coaxially to the center axis 1.

And the transmitting surfaces $21_1$, $22_1$, $21_2$ and $22_2$ in the front unit 3 are each configured such that the normal makes an angle with the center axis 1 near where it intersects the center axis 1, and in the section including the center axis 1, the transmitting optical element $2_1$, $2_2$ is configured in a substantial wedge form whose thickness in the meridional section grows large with an increasing distance from the center axis 1 to the periphery.

And in the example here, the object plane 6 is set at infinity in the Z-axis minus direction.

The rear unit 4 is made up of a cemented lens of a double-concave negative lens L1 and a double-convex positive lens L2, a cemented lens of a positive meniscus lens L3 concave on its object side and a negative meniscus lens L4 concave on its object side, a double-convex positive lens L5 and a cemented lens of a negative meniscus lens L6 convex on its object side and a double-convex positive lens L7, and the aperture stop 5 is located just before the double-concave negative lens L1. And, the image plane 7 of this optical system is in alignment with the back surface of the plane-parallel plate 9. Note here that the plane-parallel plate 9 may be dispensed with.

Such being the arrangement, an object image, having a wide angle of view of 90° at infinity, is formed as a zonal image on the planar image plane 7 vertical to the center axis 1 via the front unit 3 rotationally symmetric about the center axis 1, the stop 5 and the rear unit 4.

The specifications of Example 4 are:

Angle-of-view range: 90° (with the center angle of view of 0° ),
Entrance pupil diameter: 0.131, mm, and
Image size: $\phi$0.204 to $\phi$1.989 mm.

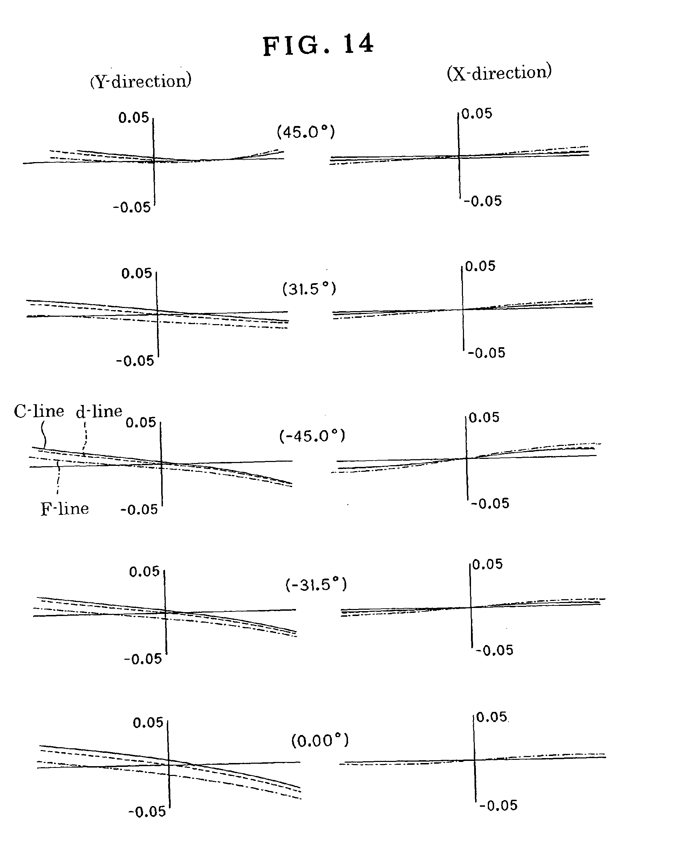

Transverse aberrations of the optical system here are shown in Fig. 14.

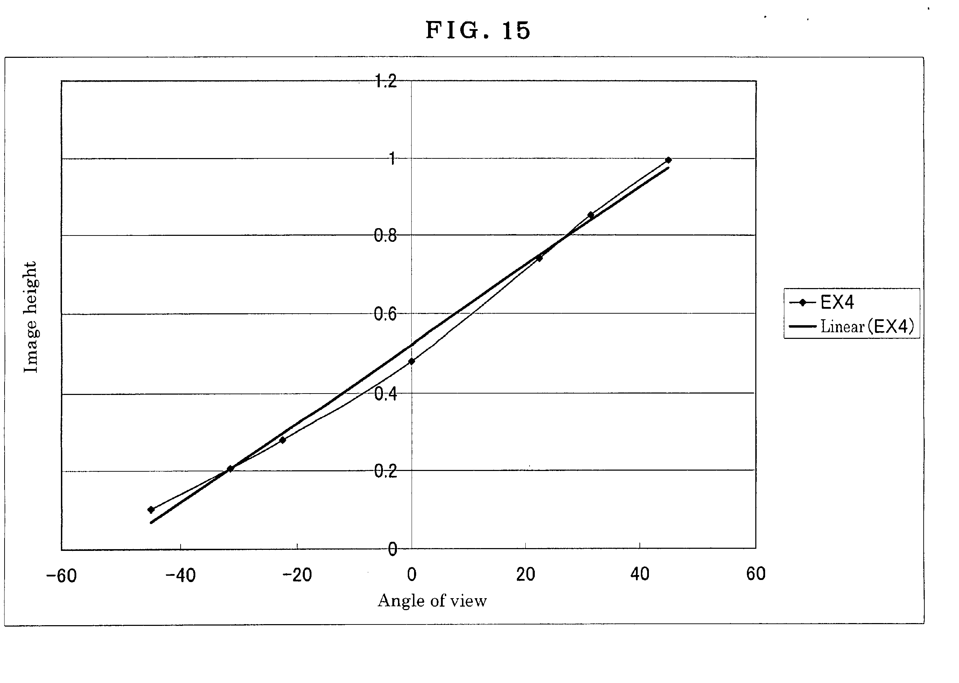

Further, angles of view (° ) vs. image height (mm) relations in the meridional section here are shown Fig. 15.

Note here that if a light block member 26 adapted to block off light rays from near the center axis 1 is located in an area of the object-side transmitting optical element $2_1$ near the center axis 1, it is then possible to prevent inessential light coming from near the center axis 1 from entering the optical system, giving rise to flares or the like.

Example 5

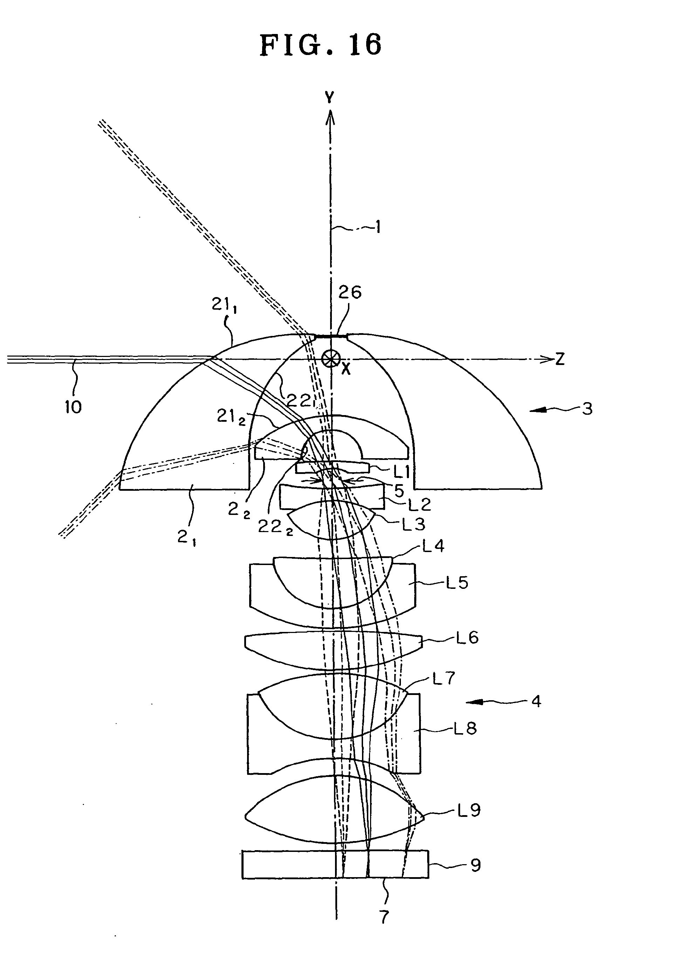

Fig. 16 is a sectional view of the optical system of Example 5 as taken along the center axis (the axis of rotational symmetry) 1.

In the optical system here, two transmitting optical elements $2_1$ and $2_2$ formed of a transparent medium having a refracting index of greater than 1 are used for the front unit 3. The first and second transmitting surfaces $21_1$ and $22_1$ of the transmitting optical element $2_1$, and the first and second transmitting surfaces $21_2$ and $22_2$ of the transmitting optical element $2_2$ are each constructed from an extended rotation free-form surface, i.e., a toric surface with the center axis 1 as the axis of rotational symmetry, which is obtained by the rotation about the center axis 1 as the axis of rotational symmetry of a curved line having only the spherical term free of any higher order term. The transmitting surfaces $21_1$, $22_1$, $21_2$ and $22_2$ are each configured such that it has a convex shape on its object side at a position where the chief ray 10 is incident and in the section including the center axis 1. The rear unit 4 that is located on the image plane 7 side with respect to the front unit 3, is rotationally symmetric about the center axis 1 and has positive power consists of six groups or nine lenses. And the aperture stop 5 is located within the rear unit 4 coaxially to the center axis 1.

And the transmitting surfaces $21_1$, $22_1$, $21_2$ and $22_2$ in the front unit 3 are each configured such that the normal makes an angle with the center axis 1 near where it intersects the center axis 1, and in the section including the center axis 1, the transmitting optical element $2_1$, $2_2$ is configured in a substantial wedge form whose thickness in the meridional section grows large with an increasing distance from the center axis 1 to the periphery.

And in the example here, the object plane 6 is set at infinity in the Z-axis minus direction.

The rear unit 4 is made up of a negative meniscus lens L1 convex on its object side, the aperture stop 5, a cemented lens of a double-concave negative lens L2 and a double-convex positive lens L3, a cemented lens of a positive meniscus lens L4 concave on its object side and a negative meniscus lens L5 concave on its object side, a double-convex positive lens L6, a cemented lens of a double-convex positive lens L7 and a double-concave negative lens L8 and a double-convex positive lens L9. And, the image plane 7 of this optical system is in alignment with the back surface of the plane-parallel plate 9. Note here that the plane-parallel plate 9 may be dispensed with.

Such being the arrangement, an object image, having a wide angle of view of 90° at infinity, is formed as a zonal image on the planar image plane 7 vertical to the center axis 1 via the front unit 3 rotationally symmetric about the center axis 1 and the rear unit 4.

The specifications of Example 5 are:

Angle-of-view range: 90° (with the center angle of view of 0°),
Entrance pupil diameter: 0.080 mm, and
Image size: $\phi$0.241 to $\phi$2.024 mm.

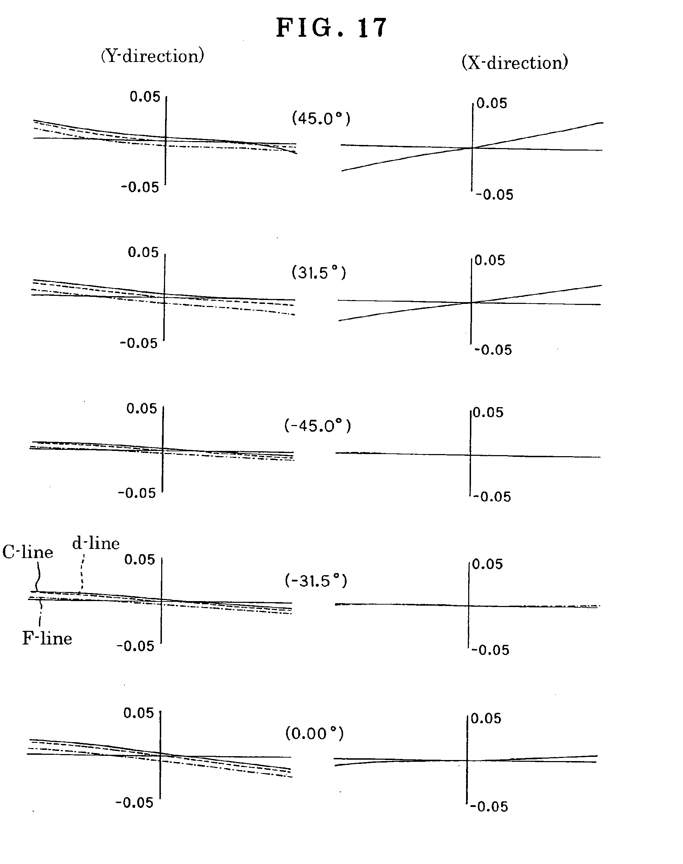

Transverse aberrations of the optical system here are shown in Fig. 17.

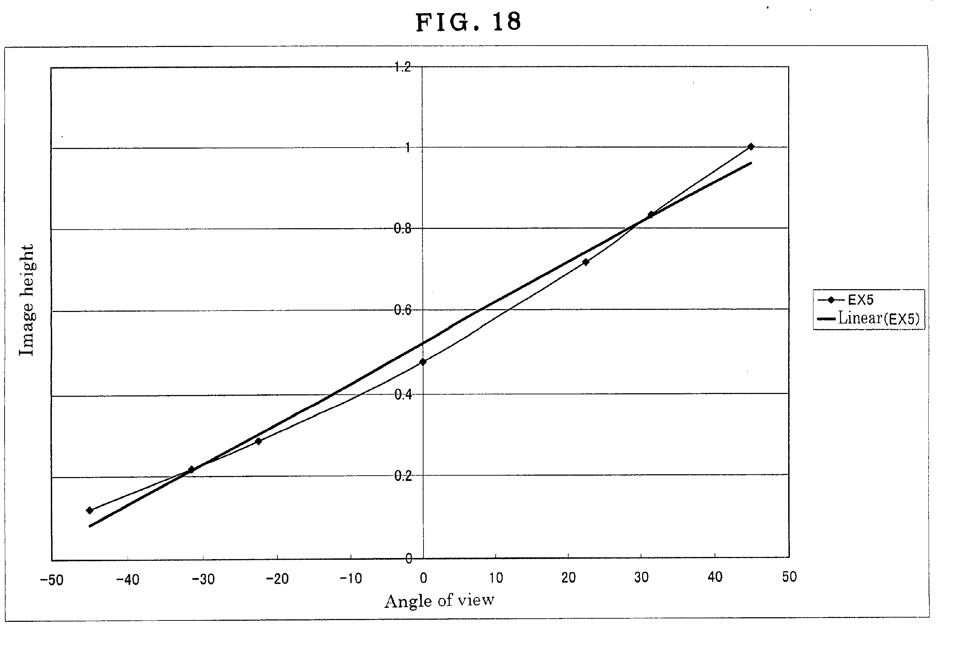

Further, angles of view (° ) vs. image height (mm) relations in the meridional section here are shown Fig. 18.

Note here that if a light block member 26 adapted to block off light rays from near the center axis 1 is located in an area of the object-side transmitting optical element $2_1$ near the center axis 1, it is then possible to prevent inessential light coming from near the center axis 1 from entering the optical system, giving rise to flares or the like.

Example 6

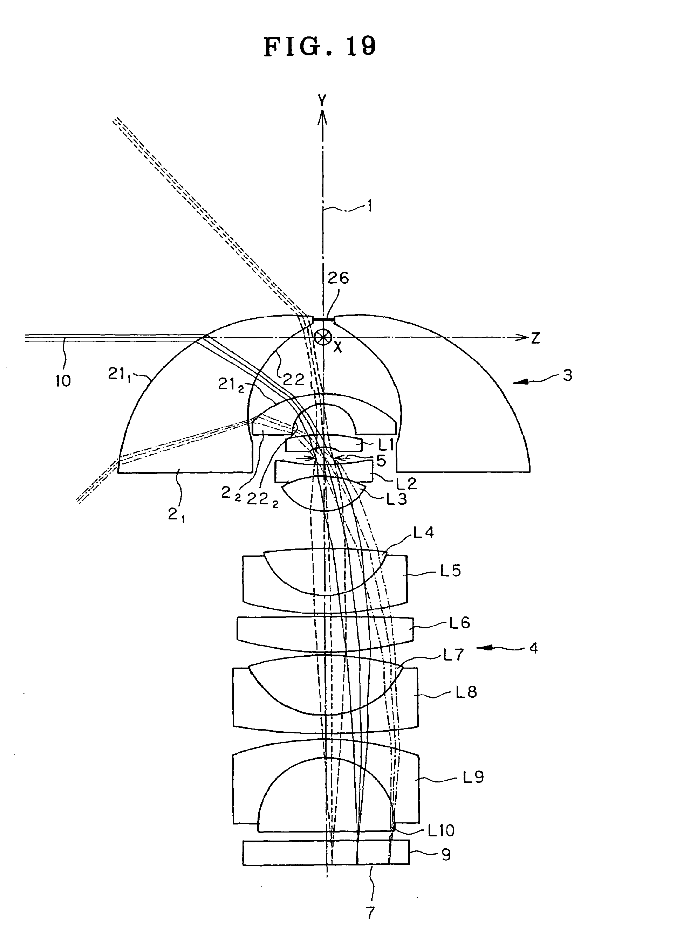

Fig. 19 is a sectional view of the optical system of Example 6 as taken along the center axis (the axis of rotational symmetry) 1.

In the optical system here, two transmitting optical elements $2_1$ and $2_2$ formed of a transparent medium having a refracting index of greater than 1 are used for the front unit 3. The first and second transmitting surfaces $21_1$ and $22_1$ of the transmitting optical element $2_1$, and the first and second transmitting surfaces $21_2$ and $22_2$ of the transmitting optical element $2_2$ are each constructed from an extended rotation free-form surface that is obtained by the rotation about the center axis 1 as the axis of rotational symmetry of a curved line with the conic coefficient and Y third order term added to the spherical term. The transmitting surfaces $21_1$, $22_1$, $21_2$ and $22_2$ are each configured such that it has a convex shape on its object side at a position where the chief ray 10 is incident and in the section including the center axis 1. The rear unit 4 that is located on the image plane 7 side with respect to the front unit 3, is rotationally symmetric about the center axis 1 and has positive power consists of six groups or ten lenses. And the aperture stop 5 is located within the rear unit 4 coaxially to the center axis 1.

And the transmitting surfaces $21_1$, $22_1$, $21_2$ and $22_2$ in the front unit 3 are each configured such that the normal makes an angle with the center axis 1 near where it intersects the center axis 1, and in the section including the center axis 1, the transmitting optical element $2_1$, $2_2$ is configured in a substantial wedge form whose thickness in the meridional section grows large with an increasing distance from the center axis 1 to the periphery.

And in the example here, the object plane 6 is set at infinity in the Z-axis minus direction.

The rear unit 4 is made up of a negative meniscus lens L1 convex on its object side, the aperture stop 5, a cemented lens of a double-concave negative lens L2 and a double-convex positive lens L3, a cemented lens of a double-convex positive lens L4 and a negative meniscus lens L5 concave on its object side, a double-convex positive lens L6, a cemented lens of a double-convex positive lens L7 and a negative meniscus lens L8 concave on its object side, and a cemented lens of a negative meniscus lens L9 concave on its image side and a positive meniscus lens L10 concave on its image side. And, the image plane 7 of this optical system is in alignment with the back surface of the plane-parallel plate 9. Note here that the plane-parallel plate 9 may be dispensed with.

Such being the arrangement, an object image, having a wide angle of view of 90° at infinity, is formed as a zonal image on the planar image plane 7 vertical to the center axis 1 via the front unit 3 rotationally symmetric about the center axis 1 and the rear unit 4.

The specifications of Example 6 are:

Angle-of-view range: 90° (with the center angle of view of 0° ),
Entrance pupil diameter: 0.097 mm, and
Image size: $\phi$0.156 to $\phi$2.009 mm.

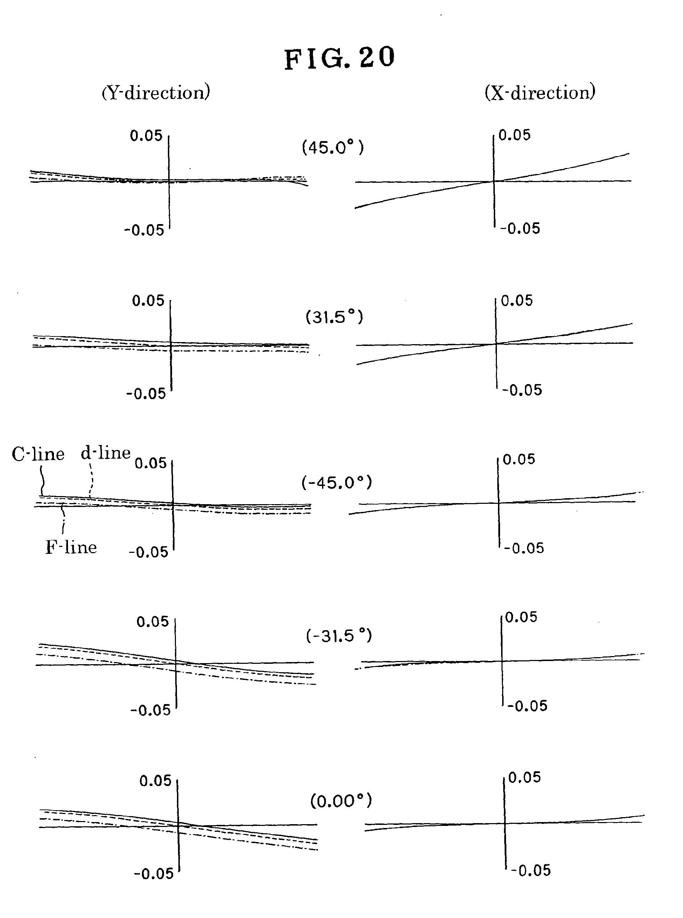

Transverse aberrations of the optical system here are shown in Fig. 20.

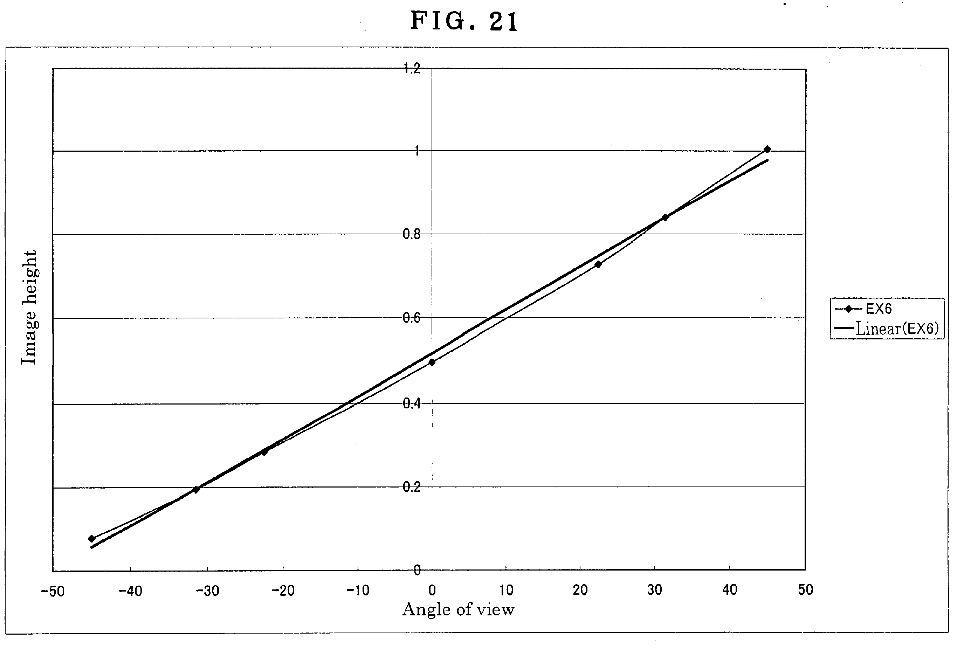

Angles of view (° ) vs. image height (mm) relations in the meridional section here are also shown Fig. 21.

Note here that if the light block member 26 adapted to block off light rays from near the center axis 1 is located in an area of the object-side transmitting optical element $2_1$ near the center axis 1, it is then possible to prevent inessential light coming from near the center axis 1 from entering the optical system, giving rise to flares or the like.

In the example here, the extended rotation free-form surface obtained by the rotation about the center axis 1 as the axis of rotational symmetry of a curved line with the conic constant and Y third order term added to the spherical term is used for the transmitting surface $21_1$, $22_1$, $21_2$, $22_2$. The Y third order term in particular, because of generating a vertically asymmetric shape with respect to the center chief ray 10, makes it possible to obtain preferable results for correction of image distortion.

Example 7

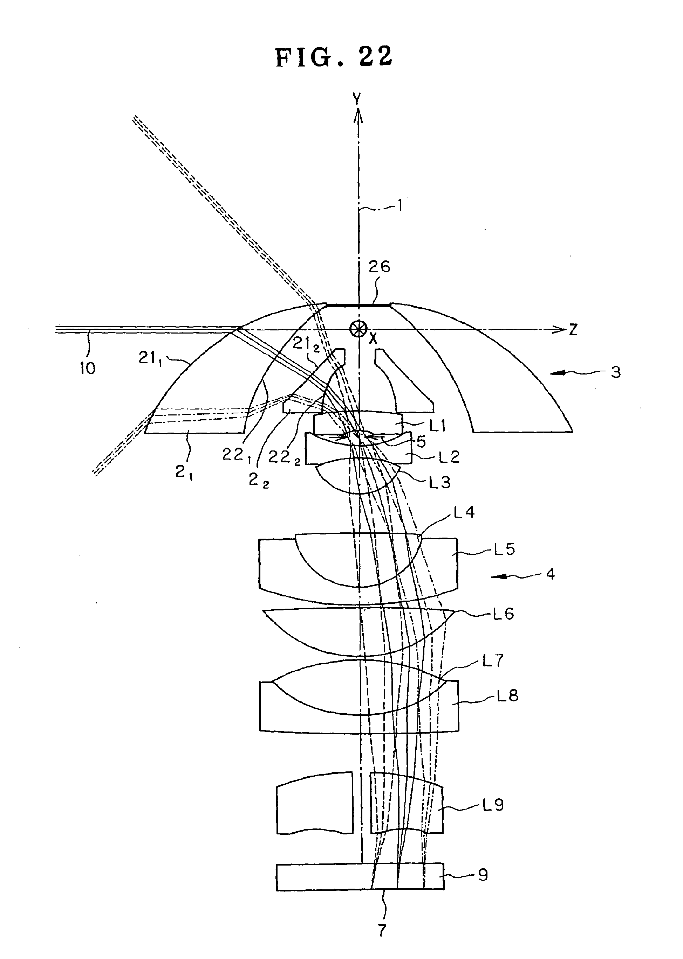

Fig. 22 is a sectional view of the optical system of Example 7 as taken along the center axis (the axis of rotational symmetry) 1.

In the optical system here, two transmitting optical elements $2_1$ and $2_2$ formed of a transparent medium having a refracting index of greater than 1 are used for the front unit 3. The first and second transmitting surfaces $21_1$ and $22_1$ of the transmitting optical element $2_1$, and the first and second transmitting surfaces $21_2$ and $22_2$ of the transmitting optical element $2_2$ are each constructed from an extended rotation free-form surface, i.e., a toric surface with the center axis 1 as the axis of rotational symmetry, which is obtained by the rotation about the center axis 1 as the axis of rotational symmetry of a curved line having only the spherical term free of any higher order term. The transmitting surfaces $21_1$, $22_1$ and $22_2$ are each configured such that it has a convex shape on its object side at a position where the chief ray 10 is incident and in the section including the center axis 1, while the transmitting surface $21_2$ is configured such that it has a concave shape at a position where the chief ray 10 is incident and in the section including the center axis 1. The rear unit 4 that is located on the image plane 7 side with respect to the front unit 3, is rotationally symmetric about the center axis 1 and has positive power consists of six groups or nine lenses. And the aperture stop 5 is located within the rear unit 4 coaxially to the center axis 1.

And the transmitting surfaces $21_1$, $22_1$, $21_2$ and $22_2$ in the front unit 3 are each configured such that the normal makes an angle with the center axis 1 near where it intersects the center axis 1, and in the section including the center axis 1, the transmitting optical element $2_1$, $2_2$ is configured in a substantial wedge form whose thickness in the meridional section grows large with an increasing distance from the center axis 1 to the periphery.

And in the example here, the object plane 6 is set at infinity in the Z-axis minus direction.

The rear unit 4 is made up of a negative meniscus lens L1 convex on its object side, the aperture stop 5, a cemented lens of a double-concave negative lens L2 and a double-convex positive lens L3, a cemented lens of a double-convex positive lens L4 and a negative meniscus lens L5 concave on its object side, a double-convex positive lens L6, a cemented lens of a double-convex positive lens L7 and a negative meniscus lens L8 concave on its object side and a positive power lens L9 comprised of an extended rotation free-form surface obtained by the rotation about the center axis 1 as the axis of rotational symmetry of a curved line having the spherical term on both surfaces, that is, a toric surface with the center axis 1 as the axis of rotational symmetry. And, the image plane 7 of this optical system is in alignment with the back surface of the plane-parallel plate 9. Note here that the plane-parallel plate 9 may be dispensed with.

Such being the arrangement, an object image, having a wide angle of view of 90° at infinity, is formed as a zonal image on the planar image plane 7 vertical to the center axis 1 via the front unit 3 rotationally symmetric about the center axis 1 and the rear unit 4.

The specifications of Example 7 are:

Angle-of-view range: 90° (with the center angle of view of 0° ),
Entrance pupil diameter: 0.083 mm, and
Image size: ϕ0.293 to ϕ2.041 mm.

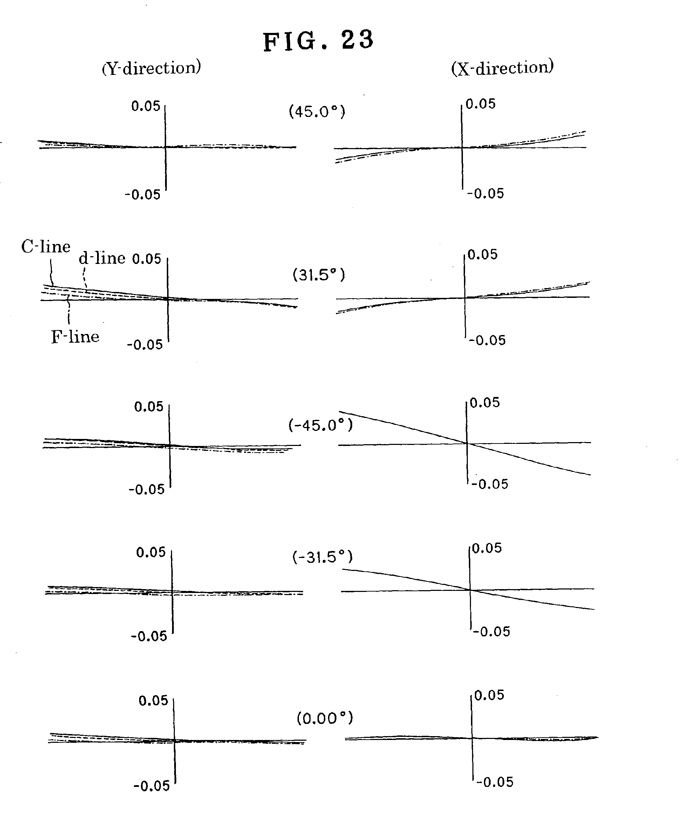

Transverse aberrations of the optical system here are shown in Fig. 23.

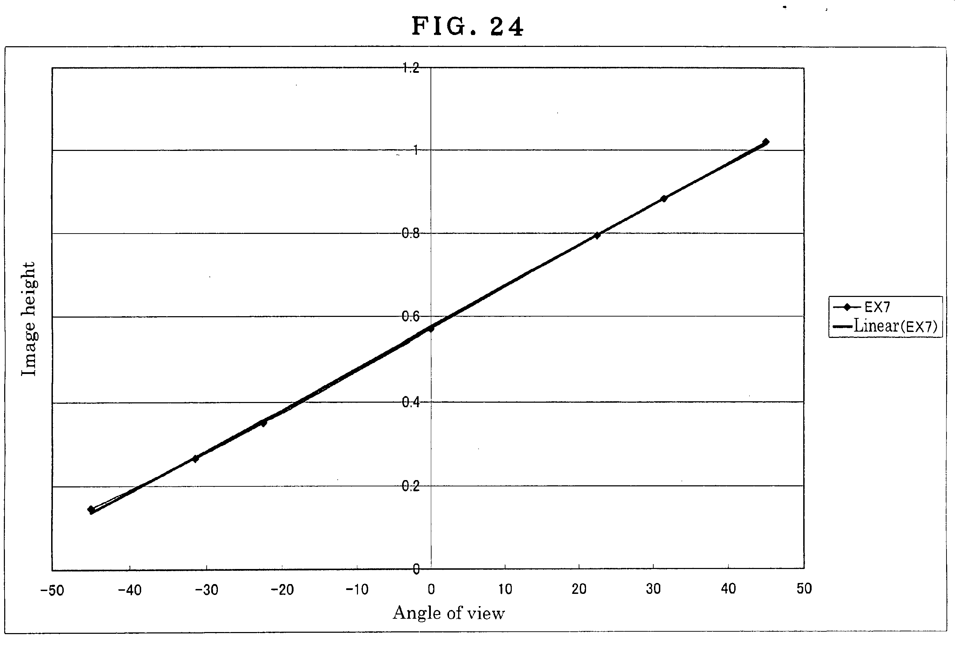

Angles of view (° ) vs. image height (mm) relations in the meridional section here are also shown Fig. 24.

Note here that if the light block member 26 adapted to block off light rays from near the center axis 1 is located in an area of the object-side transmitting optical element $2_1$ near the center axis 1, it is then possible to prevent inessential light coming from near the center axis 1 from entering the optical system, giving rise to flares or the like.

In the example here, an extended rotation free-form surface is located in the rear unit 4, too, so that astigmatism in particular can be corrected.

While the object point is at infinity in Examples 3 to 7, it is a matter of course that the image of an even more nearby object point may be taken by shifting the position of the image plane 7 in the center axis 1 direction.

The constructional parameters in Examples 1 to 7 are set out below, wherein the acronym "ERFS" indicates an extended rotation free-form surface.

Example 1

| Surface No. | | Radius of curvature | Surface separation | Displacement and tilt (1) | Refractive index | Abbe's No. |
|---|---|---|---|---|---|---|
| Object plane | | ERFS[1] | | | | |
| 1 | | ERFS[1] | | (1) | 1.5163 | 64.1 |
| 2 | | ERFS[2] | | (2) | | |
| 3 | | ERFS[3] | | (3) | 1.5163 | 64.1 |
| 4 | | ERFS[4] | | (4) | | |
| 5 | | ∞ (Stop) | 0.03 | (5) | | |
| 5 | | ∞ | 0.90 | | 1.7880 | 47.3 |
| 6 | | -1.00 | 0.10 | | | |
| 7 | | 1.70 | 0.62 | | 1.7880 | 47.3 |
| 8 | | ∞ | 0.35 | | | |
| 9 | | ∞ | 0.40 | | 1.5163 | 64.1 |
| 9 | | ∞ | 0.00 | | | |
| Image plane | | ∞ | | | | |
| | | ERFS[1] | | | | |
| RY | ∞ | | | | | |
| θ | 0.00 | | | | | |
| R | -5.50 | | | | | |
| | | ERFS[2] | | | | |
| RY | ∞ | | | | | |
| θ | 0.00 | | | | | |
| R | -4.50 | | | | | |
| | | ERFS[3] | | | | |
| RY | 12.22 | | | | | |
| θ | -64.66 | | | | | |
| R | -2.01 | | | | | |

(continued)

| Surface No. Object plane | | Radius of curvature ERFS[1] ERFS[4] | Surface separation | Displacement and tilt (1) | Refractive index | Abbe's No. |
|---|---|---|---|---|---|---|
| RY | 5.23 | | | | | |
| θ | -20.06 | | | | | |
| R | -0.70 | | | | | |
| | | Displacement and tilt(1) | | | | |
| X | 0.00 | Y | 0.00 | Z | 0.00 | |
| α | 0.00 | β | 0.00 | γ | 0.00 | |
| | | Displacement and tilt(2) | | | | |
| X | 0.00 | Y | 0.00 | Z | 0.00 | |
| α | 0.00 | β | 0.00 | γ | 0.00 | |
| | | Displacement and tilt(3) | | | | |
| X | 0. 0 | Y | -2.41 | Z | 0.00 | |
| α | 0.00 | β | 0.00 | γ | 0.00 | |
| | | Displacement and tilt(4) | | | | |
| X | 0.00 | Y | -3.85 | Z | 0.00 | |
| α | 0.00 | β | 0.00 | γ | 0.00 | |
| | | Displacement and tilt(5) | | | | |
| X | 0.00 | Y | -5. 34 | Z | 0.00 | |
| α | -90.00 | β | 0.00 | γ | 0.00 | |

Example 2

| Surface No. | Radius of curvature | Surface separation | Displacement and tilt | Refractive index | Abbe's No. |
|---|---|---|---|---|---|
| Object plane | ERFS[1] | | | | |
| 1 | ERFS[1] | | (1) | 1.5163 | 64.1 |
| 2 | ERFS[2] | | (2) | | |
| 3 | ERFS[3] | | (3) | 1.5163 | 64.1 |
| 4 | ERFS[4] | | (4) | | |
| 5 | ∞ (Stop) | 0.03 | (5) | | |
| 6 | ∞ | | 0.90 | 1.7880 | 47.3 |
| 7 | -1.00 | | 0.10 | | |
| 8 | 1.70 | | 0.62 | 1.7880 | 47.3 |
| 9 | ∞ | | 0.29 | | |
| 10 | ∞ | | 0.40 | 1.5163 | 64.1 |
| Image plane | ∞ | | | | |

| | ERFS[1] |
|---|---|
| RY | ∞ |
| θ | 0.00 |
| R | -5.50 |
| | ERFS[2] |
| RY | ∞ |
| θ | 0.00 |
| R | -4.50 |

(continued)

| Surface No. | Radius of curvature | | | | Surface separation | Displacement and tilt | Refractive index | Abbe's No. |
|---|---|---|---|---|---|---|---|---|
| | ERFS[3] | | | | | | | |
| RY | 5.41 | | | | | | | |
| θ | -45.79 | | | | | | | |
| R | -3.17 | | | | | | | |
| | ERFS[2] | | | | | | | |
| RY | 19.38 | | | | | | | |
| θ | 0.00 | | | | | | | |
| R | -1.29 | | | | | | | |
| | Displacement and tilt(1) | | | | | | | |
| X | 0.00 | Y | 0.00 | Z | 0.00 | | | |
| α | 0.00 | β | 0.00 | γ | 0.00 | | | |
| | Displacement and tilt(2) | | | | | | | |
| X | 0.00 | Y | 0.00 | Z | 0.00 | | | |
| α | 0.00 | β | 0.00 | γ | 0.00 | | | |
| | Displacement and tilt(3) | | | | | | | |
| X | 0.00 | Y | -1.13 | Z | 0.00 | | | |
| α | 0.00 | β | 0.00 | γ | 0.00 | | | |
| | Displacement and tilt(4) | | | | | | | |
| X | 0.00 | Y | -2.48 | Z | 0.00 | | | |
| α | 0.00 | β | 0.00 | γ | 0.00 | | | |
| | Displacement and tilt(5) | | | | | | | |
| X | 0.00 | Y | -4.95 | Z | 0.00 | | | |
| α | -90.00 | β | 0.00 | γ | 0.00 | | | |

Example 3

| Surface No. | Radius of curvature | Surface separation | Displacement and tilt | Refractive index | Abbe's No. |
|---|---|---|---|---|---|
| Object plane | ∞ | | (1) | | |
| 1 | 7. 91 | | (2) | 1.5163 | 64.1 |
| 2 | ERFS[1] | | (3) | | |
| 3 | ∞ (Stop) | 0.03 | (4) | | |
| 4 | ∞ | 0.30 | | 1.8052 | 25.4 |
| 5 | 0.99 | 0.60 | | 1.4875 | 70.4 |
| 6 | -0.69 | 0.10 | | | |
| 7 | 1. 04 | 0.80 | | 1.7440 | 44.9 |
| 8 | -0. 84 | 0.30 | | 1.8052 | 25.4 |
| 9 | ∞ | 0.16 | | | |
| 10 | ∞ | 0.40 | | 1.5163 | 64.1 |
| 11 | ∞ | 0.00 | | | |
| Image plane | ∞ | | | | |
| | ERFS[1] | | | | |
| RY | 1.40 | | | | |
| θ | -20.82 | | | | |
| R | -0. 73 | | | | |

(continued)

| Surface No. | Radius of curvature | | | | Surface separation | Displacement and tilt | Refractive index | Abbe's No. |
|---|---|---|---|---|---|---|---|---|
| $C_4$ | | 9.5407 × $10^{-2}$ | | | | | | |
| $C_5$ | | -1. 6486 × $10^{-2}$ | | | | | | |
| | | Displacement and tilt(1) | | | | | | |
| X | 0.00 | Y | 0.00 | Z | -∞ | | | |
| α | 0.00 | β | 0.00 | γ | 0.00 | | | |
| | | Displacement and tilt(2) | | | | | | |
| X | 0. 00 | Y | 0.16 | Z | 0.00 | | | |
| α | -90.00 | β | 0.00 | γ | 0.00 | | | |
| | | Displacement and tilt(3) | | | | | | |
| X | 0. 00 | Y | -1.06 | Z | 0.00 | | | |
| α | 0.00 | β | 0.00 | γ | 0.00 | | | |
| | | Displacement and tilt(4) | | | | | | |
| X | 0.00 | Y | -3.05 | Z | 0.00 | | | |
| α | -90.00 | β | 0.00 | γ | 0.00 | | | |

Example 4

| Surface No. | Radius of curvature | Surface separation | Displacement and tilt | Refractive index | Abbe's No. |
|---|---|---|---|---|---|
| Object plane | ∞ | | (1) | | |
| 1 | ERFS[1] | | (2) | 1.8830 | 40.7 |
| 2 | ERFS[2] | | (3) | | |
| 3 | ERFS[3] | | (4) | 1.8830 | 40.7 |
| 4 | ERFS[4] | | (5) | | |
| 5 | ∞ (Stop) | 0.030 | (6) | | |
| 6 | -2.779 | 0.200 | | 1.6146 | 46.4 |
| 7 | 0.819 | 0.600 | | 1.6234 | 59.7 |
| 8 | -0.795 | 0.151 | | | |
| 9 | -7.419 | 0.800 | | 1.6968 | 49.1 |
| 10 | -0.802 | 0.300 | | 1.8467 | 23.8 |
| 11 | -3.004 | 0.050 | | | |
| 12 | 3. 700 | 0.800 | | 1.4970 | 81.5 |
| 13 | -3.058 | 0.050 | | | |
| 14 | 2.429 | 0.300 | | 1.8467 | 23.8 |
| 15 | 1.200 | 1.000 | | 1.6414 | 56.5 |
| 16 | -14.857 | 0.321 | | | |
| 17 | ∞ | 0.400 | | 1.5163 | 64.1 |
| 18 | ∞ | 0.000 | | | |
| Image plane | ∞ | | | | |
| | ERFS[1] | | | | |
| RY | 2.812 | | | | |
| θ | -62.791 | | | | |
| R | -1.758 | | | | |
| | ERFS[2] | | | | |
| RY | 1.786 | | | | |
| θ | -28.577 | | | | |

(continued)

| Surface No. | Radius of curvature | Surface separation | Displacement and tilt | Refractive index | Abbe's No. |
|---|---|---|---|---|---|
| R | -1.002 | | | | |
| | ERFS[3] | | | | |
| RY | 2.136 | | | | |
| θ | -70.230 | | | | |
| R | -0.450 | | | | |
| | ERFS[4] | | | | |
| RY | 0.559 | | | | |
| θ | -43.461 | | | | |
| R | -0.302 | | | | |

Displacement and tilt(1)

| | | | | | |
|---|---|---|---|---|---|
| X | 0.000 | Y | 0.000 | Z | -∞ |
| α | 0.000 | β | 0.000 | γ | 0.000 |

Displacement and tilt(2)

| | | | | | |
|---|---|---|---|---|---|
| X | 0.000 | Y | 0.000 | Z | 0.000 |
| α | 0.000 | β | 0.000 | γ | 0.000 |

Displacement and tilt(3)

| | | | | | |
|---|---|---|---|---|---|
| X | 0.000 | Y | -0.524 | Z | 0.000 |
| α | 0.000 | β | 0. 000 | γ | 0.000 |

Displacement and tilt(4)

| | | | | | |
|---|---|---|---|---|---|
| X | 0.000 | Y | -0.991 | Z | 0.000 |
| α | 0.000 | β | 0.000 | γ | 0.000 |

Displacement and tilt(5)

| | | | | | |
|---|---|---|---|---|---|
| X | 0.000 | Y | -1.200 | Z | 0.000 |
| α | 0.000 | β | 0.000 | γ | 0.000 |

Displacement and tilt(6)

| | | | | | |
|---|---|---|---|---|---|
| X | 0.000 | Y | -1.858 | Z | 0.000 |
| X | -90.000 | β | 0.000 | γ | 0.000 |

Example 5

| Surface No. | Radius of curvature | Surface separation | Displacement and tilt | Refractive index | Abbe's No. |
|---|---|---|---|---|---|
| Object plane | ∞ | | (1) | | |
| 1 | ERFS[1] | | (2) | 1.8830 | 40.7 |
| 2 | ERFS[2] | | (3) | | |
| 3 | ERFS[3] | | (4) | 1.8830 | 40.7 |
| 4 | ERFS[4] | | (5) | | |
| 5 | 5.234 | 0.103 | (6) | 1.4880 | 70.3 |
| 6 | 0.400 | 0.206 | | | |
| 7 | ∞ (Stop) | 0.100 | | | |
| 8 | -5.004 | 0.200 | | 1.6302 | 47.6 |
| 9 | 1.206 | 0.600 | | 1.6212 | 40.8 |
| 10 | -0.757 | 0.290 | | | |
| 11 | -22.554 | 0.800 | | 1.7050 | 47.8 |
| 12 | -0.917 | 0.300 | | 1.8467 | 23.8 |
| 13 | -2.538 | 0.050 | | | |
| 14 | 12.200 | 0.600 | | 1.5011 | 63.1 |

(continued)

| Surface No. | Radius of curvature | Surface separation | Displacement and tilt | Refractive index | Abbe's No. |
|---|---|---|---|---|---|
| 15 | -2.750 | 0.050 | | | |
| 16 | 2.492 | 1.000 | | 1.5966 | 60.8 |
| 17 | -1.261 | 0.300 | | 1.8467 | 23.8 |
| 18 | 1.982 | 0.267 | | | |
| 19 | 1.838 | 1.000 | | 1.4877 | 70.3 |
| 20 | -2.787 | 0.100 | | | |
| 21 | ∞ | 0.400 | | 1.5163 | 64.1 |
| 22 | ∞ | 0.000 | | | |
| Image plane | ∞ | | | | |

| | ERFS[1] |
|---|---|
| RY | 2.948 |
| θ | -60.333 |
| R | -1.843 |
| | ERFS[2] |
| RY | 2.061 |
| θ | -26.667 |
| R | -1.041 |
| | ERFS[3] |
| RY | 1.684 |
| θ | -71.813 |
| R | -0.501 |
| | ERFS[4] |
| RY | 0.432 |
| θ | -45.199 |
| R | -0.315 |

| | | | Displacement and tilt(1) | | |
|---|---|---|---|---|---|
| X | 0.000 | Y | 0.000 | Z | -∞ |
| α | 0.000 | β | 0.000 | γ | 0.000 |
| | | | Displacement and tilt(2) | | |
| X | 0.000 | Y | 0.000 | Z | 0.000 |
| α | 0.000 | β | 0.000 | γ | 0.000 |
| | | | Displacement and tilt(3) | | |
| X | 0.000 | Y | -0.536 | Z | 0.000 |
| α | 0.000 | β | 0.000 | γ | 0.000 |
| | | | Displacement and tilt(4) | | |
| X | 0.000 | Y | -0.951 | Z | 0.000 |
| α | 0.000 | β | 0.000 | γ | 0.000 |
| | | | Displacement and tilt(5) | | |
| X | 0.000 | Y | -1.213 | Z | 0.000 |
| α | 0.000 | β | 0.000 | γ | 0.000 |
| | | | Displacement and tilt(6) | | |
| X | 0.000 | Y | -1.600 | Z | 0.000 |
| α | -90.000 | β | 0.000 | γ | 0.000 |

Example 6

| Surface No. | Radius of curvature | Surface separation | Displacement and tilt | Refractive index | Abbe's No. |
|---|---|---|---|---|---|
| Object plane | ∞ | | (1) | | |
| 1 | ERFS[1] | | (2) | 1.8830 | 40.7 |
| 2 | ERFS[2] | | (3) | | |
| 3 | ERFS[3] | | (4) | 1.8830 | 40.7 |
| 4 | ERFS[4] | | (5) | | |
| 5 | 2.908 | 0.200 | (6) | 1.5163 | 64.1 |
| 6 | 0.387 | 0.200 | | | |
| 7 | ∞ (Stop) | 0.100 | | | |
| 8 | -4.520 | 0.200 | | 1.6764 | 31.7 |
| 9 | 1.391 | 0.600 | | 1.6436 | 37.5 |
| 10 | -0.759 | 0.635 | | | |
| 11 | 8.819 | 0.800 | | 1.7412 | 45.0 |
| 12 | -1.073 | 0.300 | | 1.8467 | 23.8 |
| 13 | -5.075 | 0.050 | | | |
| 14 | 62.541 | 0.600 | | 1.4970 | 81.5 |
| 15 | -5.306 | 0.050 | | | |
| 16 | 4.493 | 1.000 | | 1.6750 | 51.6 |
| 17 | -1.426 | 0.300 | | 1.8467 | 23.8 |
| 18 | -10.502 | 0.103 | | | |
| 19 | 4.823 | 0.300 | | 1.6014 | 40.1 |
| 20 | 1.121 | 1.200 | | 1.6183 | 60.3 |
| 21 | 426.799 | 0.130 | | | |
| 22 | ∞ | 0.400 | | 1.5163 | 64.1 |
| 23 | ∞ | 0.000 | | | |
| Image plane | ∞ | | | | |

| | ERFS[1] |
|---|---|
| RY | 2.889 |
| θ | -60.517 |
| R | -2.023 |
| $C_1$ | $-1.8603 \times 10^{-2}$ |
| $C_4$ | $-2.3444 \times 10^{-3}$ |
| | ERFS[2] |
| RY | 1.581 |
| θ | -29.554 |
| R | -1.084 |
| $C_1$ | $-2.9790 \times 10^{-1}$ |
| $C_4$ | $-7.2760 \times 10^{-2}$ |
| | ERFS[3] |
| RY | 1.565 |
| θ | -72.421 |
| R | -0.525 |
| $C_1$ | $-8.1208 \times 10^{-1}$ |
| $C_4$ | $-1.2750 \times 10^{-3}$ |
| | ERFS[4] |
| RY | 0.490 |
| θ | -45. 951 |
| R | -0.361 |
| $C_1$ | $2.5646 \times 10^{-2}$ |

(continued)

| Surface No. | Radius of curvature | | | | Surface separation | Displacement and tilt | Refractive index | Abbe's No. |
|---|---|---|---|---|---|---|---|---|
| $C_4$ | | -2.7849 $\times 10^{-1}$ | | | | | | |
| | Displacement and tilt(1) | | | | | | | |
| X | 0.000 Y | 0.000 | Z | -∞ | | | | |
| α | 0.000 β | 0.000 | γ | 0.000 | | | | |
| | Displacement and tilt(2) | | | | | | | |
| X | 0.000 Y | 0.000 | Z | 0.000 | | | | |
| α | 0.000 β | 0.000 | γ | 0.000 | | | | |
| | Displacement and tilt(3) | | | | | | | |
| X | 0.000 Y | -0.611 | Z | 0.000 | | | | |
| α | 0.000 β | 0.000 | γ | 0.000 | | | | |
| | Displacement and tilt(4) | | | | | | | |
| X | 0.000 Y | -1.021 | Z | 0.000 | | | | |
| α | 0.000β | 0. 000 | γ | 0.000 | | | | |
| | Displacement and tilt(5) | | | | | | | |
| X | 0.000 Y | -1. 247 | Z | 0.000 | | | | |
| α | 0.000β | 0.000 | γ | 0.000 | | | | |
| | Displacement and tilt(6) | | | | | | | |
| | X | 0.000 | Y | -1. 627 | Z | 0.000 | | |
| | α | -90.000 | β | 0.000 | γ | 0.000 | | |

Example 7

| Surface No. | Radius of curvature | Surface separation | Displacement and tilt | Refractive index | Abbe's No. |
|---|---|---|---|---|---|
| Object plane | ∞ | | (1) | | |
| 1 | ERFS[1] | | (2) | 1.8830 | 40.7 |
| 2 | ERFS[2] | | (3) | | |
| 3 | ERFS[3] | | (4) | 1.8830 | 40.7 |
| 4 | ERFS[4] | | (5) | | |
| 5 | 3.542 | 0.326 | (6) | 1.5025 | 62.2 |
| 6 | 0. 368 | 0.159 | | | |
| 7 | ∞ (Stop) | 0. 100 | | | |
| 8 | -1. 448 | 0. 200 | | 1.6824 | 36.7 |
| 9 | 1. 657 | 0. 600 | | 1.6709 | 34.6 |
| 10 | -0. 723 | 0.655 | | | |
| 11 | 31. 831 | 0. 900 | | 1.6758 | 49.9 |
| 12 | -1. 046 | 0. 300 | | 1.8467 | 23.8 |
| 13 | -4. 841 | 0.050 | | | |
| 14 | 32.179 | 0. 800 | | 1.4878 | 70.2 |
| 15 | -1.998 | 0. 050 | | | |
| 16 | 3.189 | 0. 900 | | 1.4955 | 65.7 |
| 17 | -2.104 | 0. 300 | | 1.8467 | 23.8 |
| 18 | -26.406 | *0. 676 | | | |
| 19 | ERFS[5] | | (7) | 1.6426 | 37.1 |
| 20 | ERFS[6] | | (8) | | |
| 21 | ∞ | 0.400 | | 1.5163 | 64.1 |
| 22 | ∞ | 0. 000 | | | |

(continued)

| Surface No. | Radius of curvature | Surface separation | Displacement and tilt | Refractive index | Abbe's No. |
|---|---|---|---|---|---|
| Image plane | ∞ | | | | |

(*) The surface spacing for the 18th surface 0.676 is a spacing between the 18th surface and the 21st surface.

| | ERFS[1] |
|---|---|
| RY | 3.636 |
| θ | -61.252 |
| R | -1.960 |
| | ERFS[2] |
| RY | 3.583 |
| θ | -35.359 |
| R | -1.305 |
| | ERFS[3] |
| RY | -8.819 |
| θ | -43.922 |
| R | -0.759 |
| | ERFS[4] |
| RY | 0.929 |
| θ | -20.488 |
| R | -0.512 |
| | ERFS[5] |
| RY | 3.544 |
| θ | -100.214 |
| R | 0.737 |
| | ERFS[6] |
| RY | 1.293 |
| θ | - 89.033 |
| R | 0.676 |

Displacement and tilt(1)

| | | | | | |
|---|---|---|---|---|---|
| X | 0.000 | Y | 0. 000 | Z | -∞ |
| α | 0.000 | β | 0.000 | γ | 0.000 |

Displacement and tilt(2)

| | | | | | |
|---|---|---|---|---|---|
| X | 0.000 | Y | 0.000 | Z | 0.000 |
| α | 0.000 | β | 0.000 | γ | 0.000 |

Displacement and tilt(3)

| | | | | | |
|---|---|---|---|---|---|
| X | 0.000 | Y | -0.435 | Z | 0.000 |
| α | 0.000 | β | 0.000 | γ | 0.000 |

Displacement and tilt(4)

| | | | | | |
|---|---|---|---|---|---|
| X | 0.000 | Y | -0.776 | Z | 0.000 |
| α | 0.000 | β | 0.000 | γ | 0.000 |

Displacement and tilt(5)

| | | | | | |
|---|---|---|---|---|---|
| X | 0.000 | Y | -0.966 | Z | 0.000 |
| α | 0.000 | β | 0.000 | γ | 0.000 |

Displacement and tilt(6)

| | | | | | |
|---|---|---|---|---|---|
| X | 0.000 | Y | -1.345 | Z | 0.000 |
| α | -90.000 | β | 0.000 | γ | 0.000 |

Displacement and tilt(7)

| | | | | | |
|---|---|---|---|---|---|
| X | 0.000 | Y | -7.368 | Z | 0.000 |
| α | 0.000 | β | 0.000 | γ | 0.000 |

Displacement and tilt(8)

| | | | | | |
|---|---|---|---|---|---|
| X | 0.000 | Y | -8.209 | Z | 0.000 |

(continued)

| Surface No. | Radius of curvature | | | | Surface separation | Displacement and tilt | Refractive index | Abbe's No. |
|---|---|---|---|---|---|---|---|---|
| α | 0.000 | β | 0.000 | γ | 0.000 | | | |

In Example 1, and 2 mentioned above, the surface obtained by the rotation about the center axis 1 of the curved line defined by an arc has been used for the first 21, and the second transmitting surface 22 of the transmitting optical element 2; however, it is understood that use may be made of an extended rotation free-form surface obtained by the rotation about the center axis 1 of an arc curve having a higher order term or, alternatively, that surface may be replaced by a curved surface of any desired shape. Likewise, in Example 4, 5, and 7, the surface obtained by the rotation about the center axis 1 of the curved line defined by an arc has been used for each of the two transmitting surfaces of the transmitting optical element $2_1$, $2_2$; however, it is understood that use may be made of an extended rotation free-form surface obtained by the rotation about the center axis 1 of an arc curve having a higher order term as is the case with the transmitting optical element $2_1$, $2_2$ in Example 6 or, alternatively, that surface may be replaced by a curved surface of any desired shape.

Further for the inventive optical system, it is possible to add one Y toric lens (that is a lens having a lens surface constructed from a toric surface with the center axis 1 as the axis of rotational symmetry) to a further object side of the front unit 3. In this case, too, that lens may be constructed of a rotationally symmetric surface with the respect to the center axis 1. With that lens, it is possible to achieve a larger angle of view in the meridional section direction, because it has no concentric power in the sagittal direction but has negative power in the meridional direction.

More preferably, that lens should be constructed of a negative meniscus lens convex on its object side in the meridional section, because the occurrence of image distortion can be minimized to make sure good aberration correction.

Yet further, just only one meniscus negative lens but also two or three meniscus lenses may be located on the object side of the inventive optical system, thereby holding back the occurrence of image distortion.

By immediate use of the transmitting optical element 2, $2_1$, $2_2$ according to the invention, images having a full 360° -direction angle of view may be taken or projected. However, it is acceptable to cut that transmitting optical element 2 in a section including the center axis 1 into 1/2, 1/3, 2/3, etc. for the purpose of taking or projecting an image having an angle of view of 180° , 120° , 240° , etc. about the center axis 1.

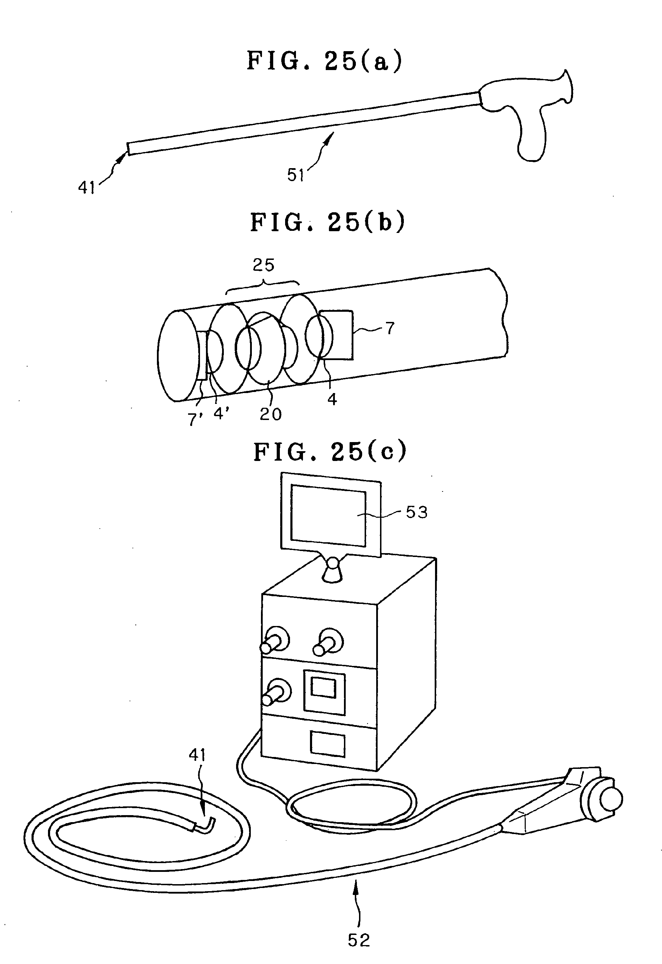

In what follows, an account will be given of an example of using a wide angle-of-view taking optical system 41 as an exemplary application of the inventive optical system. Fig. 25 is illustrative of using the inventive wide angle-of-view taking optical system 41 as a taking optical system at the distal end of an endoscope: Fig. 25(a) is illustrative of mounting the inventive wide angle-of-view taking optical system 41 on the distal end of a hard endoscope 51 to take and observe wide angle-of-view images in a full 360° -direction, and Fig. 25(b) is illustrative in schematic of the arrangement of that end. In the example here, the common transmitting optical element 20 of Fig. 8 is used, and two such optical systems as exemplified in Example 2 are incorporated in the distal end. The center axis of that optical system 41 is located along the lengthwise direction of the distal end of the hard endoscope 51, and a transparent window 25 all over the full 360° -circumference is formed in a cover at the distal end of the hard endoscope 51 so that light from objects around that window 25 enters the optical system via the window 25 to take object images by imaging devices located at the image planes 7 and 7'.

Fig. 25(c) is illustrative of an example of mounting the inventive wide angle-of-view taking optical system 41 on the distal end of a soft electronic endoscope 52 in a similar way to display taken images on a display 53 wherein the taken images are corrected for distortion by image processing.

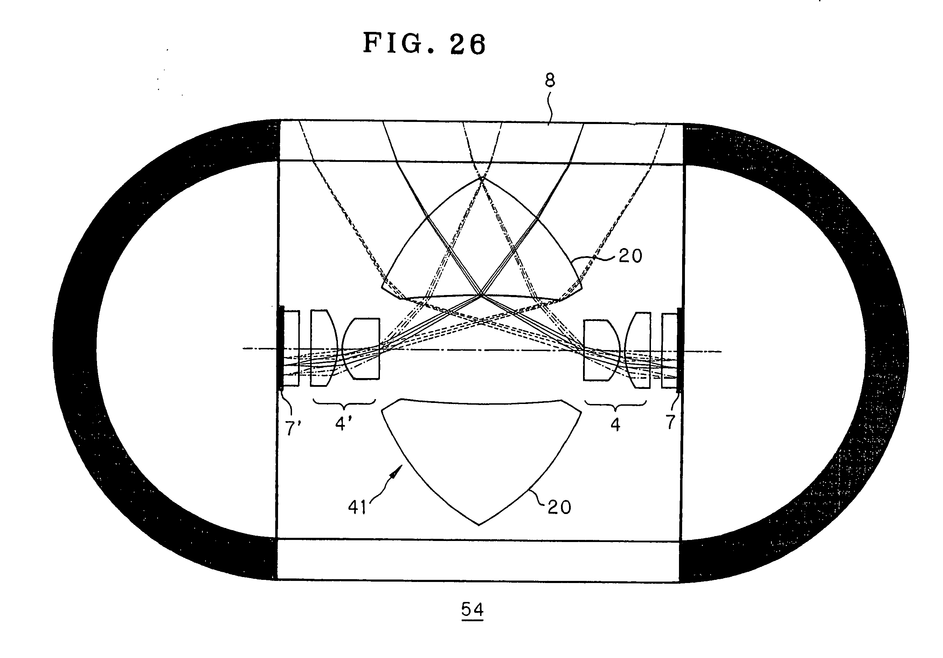

Fig. 26 is illustrative of an example of using the inventive wide angle-of-view taking optical system 41 as a taking optical system for a capsule endoscope 54. In the example here, the common transmitting optical element 20 of Fig. 8 is used, and two such optical systems as exemplified in Example 2 are incorporated inside. The center axis of that optical system 41 is located substantially parallel with the lengthwise center axis of the capsule endoscope 54, and a transparent cylinder 8 is provided all over the lengthwise side of the capsule endoscope 54 so that light from objects in the full-360° direction, for instance, the intestinal wall in close contact with that transparent cylinder 8, enters the optical system 41 via that transparent cylinder 8 to take by imaging devices located at the image plane 7 and 7' and observe wide angle-of-view images in the full-360° direction, for instance, the intestinal wall. In the example here, the transparent cylinder 8 constitutes part of the transparent cover on the side of the capsule endoscope 54.

APPLICABILITY TO THE INDUSTRY

The invention as described above makes it possible to obtain a transparent optical element that is of small-format size, is well corrected for aberrations, has high resolving power, and is capable of taking or projecting wide angle-of-view images in a simplified arrangement.

## Claims

1. A transmitting optical element comprising a transparent medium including two transmitting surfaces rotationally symmetric about a center axis and having a refractive index of greater than 1, **characterized in that** at least one transmitting surface is configured such that a normal thereto makes an angle with the center axis near where the center axis intersects said transmitting surface.

2. An optical system that is an imaging system adapted to form an object around a center axis on a planar image plane orthogonal to the center axis, **characterized by** comprising a front unit rotationally symmetric about the center axis and a rear unit that is rotationally symmetric about the center axis and has positive power, wherein said front unit comprises at least one transmitting optical element formed of a transparent medium comprising two transmitting surfaces rotationally symmetric about the center axis and having a refractive index of greater than 1, wherein at least one transmitting surface of said transmitting optical element is configured such that a normal thereto makes an angle with the center axis near where the center axis intersects said transmitting surface.

3. The optical system according to claim 2, **characterized in that** between said front unit and said rear unit or in said rear unit, there is an aperture provided coaxially to the center axis.

4. The optical system according to claim 3,
**characterized in that** any of said transmitting surfaces of said transmitting optical element is configured in such a way as to be concave with respect to said aperture.

5. The optical system according to claim 3 or 4, **characterized by** satisfying the following condition (1):

$$1^\circ < \theta < 90^\circ \quad \cdots (1)$$

where tangents of the two transmitting surfaces of said transmitting optical element make an angle θ (° ) at a point where a chief ray strikes a first transmitting surface located in said front unit and nearest to an object side and a point where a chief ray strikes a second transmitting surface with the proviso that the chief ray is defined by a ray that travels from a center of an angle of view in a direction along the center axis through a center of said aperture.

6. The optical system according to any one of claims 2 to 4, **characterized in that** at least said transmitting optical element located in said front unit and nearest to an object side comprises a light block member adapted to block off rays from on the center axis.

7. The optical system according to any one of claims 2 to 4, **characterized by** forming an image around a cylindrical or spherical full-360° circumference on a two-dimensional imaging device located on said image plane.

8. The optical system according to any one of claims 2 to 4, **characterized in that** said transmitting surface configured such that the normal thereto makes an angle with the center axis near where it intersects the center axis has a rotationally symmetric shape that is formed by rotation about the center axis of a curved line of any desired shape having no plane of symmetry.

9. The optical system according to any one of claims 2 to 4, **characterized in that** said transmitting surface configured such that the normal thereto makes an angle with the center axis near where it intersects the center axis have a rotationally symmetric shape that is formed by rotation about the center axis of a curved line of any desired shape including an odd order term.

10. The optical system according to any one of claims 2 to 4, **characterized by** being used as an optical system adapted to project an image placed on an image plane onto an object plane.

11. An endoscope, **characterized by** comprising an optical system as recited in any one of claims 2 to 4.

12. A capsule endoscope, **characterized by** comprising an optical system as recited in any one of claims 2 to 4.

13. An endoscope, **characterized by** comprising:

an optical system as recited in any one of claims 2 to 4 at a distal end, wherein:
said optical system has a center axis located along a lengthwise direction of the distal end of said endoscope,
a side of said distal end has a transparent portion all over a full circumference, and
light from an object enters said optical system via said transparent portion.

**14.** A capsule endoscope, **characterized by** comprising:

an optical system as recited in any one of claims 2 to 4 inside, wherein:
said optical system has a center axis located substantially parallel with a lengthwise center axis of said capsule endoscope,
a lengthwise side of said capsule endoscope has a transparent portion all over a full circumference, and
light from an object enters said optical system via said transparent portion.

# FIG. 1

1
Y
X
Z
6
11
8
12
10
21
21
22
22
2
3
5
L1
4
L2
9
7

# FIG. 2

(Y-direction)
(X-direction)
0.025
-0.025
(-1.00 )
(-0.50 )
(1.00 )
(0.50 )
(0.00 )
C-line
d-line
F-line

FIG. 3

EX1
Linear (EX1)
Image height
Object height
0.9
0.8
0.7
0.6
0.5
0.4
0.3
0.2
0.1
0
−5
−4
−3
−2
−1
0
1
2
3
4
5

FIG. 4

1
Y
X
Z
6
11
12
8
10
21
23
21
24
22
22
2
3
5
L1
4
L2
9
7

FIG. 5

1
Y
13
Z
X
21
21
6
12
22
22
11
8
2
2
3
5
L1
4
L2
9
7

# FIG. 6

(Y-direction)
(X-direction)
0.025
-0.025
(-1.00 )
(-0.50 )
(1.00 )
(0.50 )
(0.00 )
C-line
d-line
F-line

FIG. 7
EX2
Linear(EX2)
Image height
Object height
0.7
0.6
0.5
0.4
0.3
0.2
0.1
0
-2.5
-2
-1.5
-1
-0.5
0
0.5
1
1.5
2
2.5

# FIG. 8

7'
4'
20
13
2
6
1
6'
2'
8
13'
4
7

FIG. 9

13
7
6
4
8
3
2
14
1

FIG. 10
Z
Y
X
1
2
3
4
5
7
9
10
21
22
22
L1
L2
L3
L4

# FIG. 11

(Y-direction)
(X-direction)
0.025
-0.025
(0.00°)
(-16.3°)
(-65.0°)
(-48.8°)
(-32.5°)
d-line
C-line
F-line

FIG. 12

EX3
Linear(EX3)
Image height
Angle of view
0.5
0.45
0.4
0.35
0.3
0.25
0.2
0.15
0.1
0.05
0
-70
-60
-50
-40
-30
-20
-10
0

# FIG. 13

Y
1
26
$21_1$
X
Z
10
22
3
$21_2$
5
$2_2$
L1
$22_2$
L2
L3
$2_1$
L4
L5
4
L6
L7
9
7

FIG. 14

(Y-direction) (X-direction)

0.05 (45.0°) 0.05
-0.05 -0.05

0.05 (31.5°) 0.05
-0.05 -0.05

C-line d-line 0.05 (-45.0°) 0.05
F-line -0.05 -0.05

0.05 (-31.5°) 0.05
-0.05 -0.05

0.05 (0.00°) 0.05
-0.05 -0.05

# FIG. 15

EX4
Linear (EX4)
Image height
Angle of view
1.2
1
0.8
0.6
0.4
0.2
0
-60
-40
-20
0
20
40
60

FIG. 16

Y
1
26
$21_1$
Z
X
10
$22_1$
3
$21_2$
L1
5
L2
$2_2$
L3
$22_2$
$2_1$
L4
L5
L6
L7
4
L8
L9
9
7

FIG. 17

(Y-direction)

(X-direction)

0.05

(45.0°)

0.05

-0.05

-0.05

0.05

(31.5°)

0.05

-0.05

-0.05

0.05

(-45.0°)

0.05

-0.05

-0.05

C-line

d-line

0.05

(-31.5°)

0.05

F-line

-0.05

-0.05

0.05

(0.00°)

0.05

-0.05

-0.05

FIG. 18

Image height
1.2
1
0.8
0.6
0.4
0.2
0
-50
-40
-30
-20
-10
0
10
20
30
40
50
Angle of view
EX5
Linear(EX5)

FIG. 19

Y
1
26
Z
X
10
$21_1$
$21_2$
22
3
L1
5
L2
L3
$2_2$
$22_2$
$2_1$
L4
L5
L6
4
L7
L8
L9
L10
9
7

# FIG. 20

(Y-direction)
(X-direction)
0.05
-0.05
(45.0°)
(31.5°)
(-45.0°)
(-31.5 °)
(0.00°)
C-line
d-line
F-line

FIG. 21
Image height
Angle of view
EX6
Linear(EX6)
1.2
1
0.8
0.6
0.4
0.2
0
-50
-40
-30
-20
-10
0
10
20
30
40
50

FIG. 22

# FIG. 23

(Y-direction)
(X-direction)
0.05
-0.05
(45.0°)
C-line
d-line
F-line
(31.5°)
(-45.0°)
(-31.5°)
(0.00°)

FIG. 24

1.2
1
0.8
0.6
0.4
0.2
0
-50
-40
-30
-20
-10
0
10
20
30
40
50
Angle of view
Image height
EX7
Linear(EX7)

FIG. 25(a)
41
51
FIG. 25(b)
25
7
7'
4'
20
4
FIG. 25(c)
53
41
52

8
20
7
4
20
54
41
4'
7'

FIG. 26

## INTERNATIONAL SEARCH REPORT

International application No.
PCT/JP2007/060309

A. CLASSIFICATION OF SUBJECT MATTER
*G02B13/04*(2006.01)i, *A61B1/00*(2006.01)i, *G02B13/18*(2006.01)i, *G02B17/08*(2006.01)i, *G02B23/26*(2006.01)i

According to International Patent Classification (IPC) or to both national classification and IPC

B. FIELDS SEARCHED

Minimum documentation searched (classification system followed by classification symbols)
G02B13/04, A61B1/00, G02B13/18, G02B17/08, G02B23/26

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 11-6904 A (Reax Co., Ltd.),<br>12 January, 1999 (12.01.99),<br>Claims 1 to 6; examples; Figs. 1 to 11<br>(Family: none) | 1-7,10,11,13<br>8,9,12,14 |
| X<br>Y | JP 2006-251747 A (Olympus Corp.),<br>21 September, 2006 (21.09.06),<br>Par. Nos. [0111] to [0117]; examples 2, 3;<br>Figs. 6 to 23<br>& US 2006/0114576 A1 | 1-3,5-11,13<br>12,14 |
| X<br>Y | JP 2006-154365 A (Olympus Corp.),<br>15 June, 2006 (15.06.06),<br>Examples 2 to 4; Figs. 3 to 8<br>(Family: none) | 1-3,5-7,10<br>8,9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 August, 2007 (08.08.07) | 21 August, 2007 (21.08.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2007/060309

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2001-174713 A (Asahi Optical Co., Ltd.), 29 June, 2001 (29.06.01), Par. Nos. [0001], [0002] (Family: none) | 12,14 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2001174713 A
- US 5604531 A